# EUROPEAN PATENT APPLICATION

(11) **EP 4 458 352 A1**
(43) Date of publication of application: **06.11.2024**
(21) Application number: 23171380.1
(22) Date of filing: 03.05.2023
(51) Int. Cl.: A61K 31/232, A61P 3/04, A61K 45/06

(54) **EICOSAPENTAENOIC ACID ESTER DERIVATIVES FOR THE TREATMENT OF OBESITY**

(71) Applicant: Technische Universität Dresden, 01069 Dresden (DE)
(72) Inventor: Alexaki, Vasileia Ismini, 01309 Dresden (DE); Witt, Anke, 01307 Dresden (DE); Mateska, Ivona, 01309 Dresden (DE)
(74) Representative: Hertin und Partner Rechts- und Patentanwälte PartG mbB

(57) **Abstract**

The invention relates to an eicosapentaenoic acid (EPA) ester derivative for use in the prevention and/or treatment of obesity in a human subject. The invention also relates to a method for treating obesity in a human subject, preferably central obesity, wherein the subject is treated with an EPA ester derivative. The invention also relates to a method for reducing cortisol levels in a human subject, wherein the subject is administered with an EPA ester derivative. The invention further relates to a pharmaceutical composition comprising an EPA ester derivative with one or more pharmaceutical excipients for use in the prevention and/or treatment of obesity in a human subject. In embodiments, the subject has levels of cortisol above a reference value and/or above an average cortisol level of healthy human subjects.

## Description

The present invention is in the field of pharmaceutical substances and compositions, and their corresponding medical use.

In one aspect, the invention relates to an eicosapentaenoic acid (EPA) ester derivative for use in the prevention and/or treatment of obesity in a human subject. In embodiments, the eicosapentaenoic acid (EPA) ester derivative is an eicosapentaenoic acid ethyl ester (icosapent ethyl).

In another aspect, the invention relates to a method for treating obesity in a human subject, preferably central obesity, wherein the subject is treated with an EPA ester derivative.

In a further aspect, the invention relates to a method for reducing cortisol levels in a human subject, wherein the subject is administered with an EPA ester derivative.

In a further aspect, the invention relates to use of an EPA ester derivative for the manufacture of a medicament for the prevention and/or treatment of obesity.

The invention further relates to a pharmaceutical composition comprising an EPA ester derivative with one or more pharmaceutical excipients for use in the prevention and/or treatment of obesity in a human subject.

In embodiments, the subject has levels of cortisol, preferably in a hair sample or a blood sample, more preferably a plasma sample, above a reference value and/or above an average cortisol level of healthy human subjects.

### BACKGROUND OF THE INVENTION

Overweight and obesity have increased sharply in recent years, especially in industrialized countries. Rising obesity rates of well over 20 percent represent a serious burden on national healthcare systems. The costs caused by obesity, in addition to direct treatment and drug costs, indirect costs such as loss of production or premature retirement must also be taken into account, are already estimated to account for 5 to 15 percent of the total health care costs of western industrialized countries.

Obesity is associated with hyperfunction of the adrenal cortex and thus increased production of glucocorticoids (stress hormones). Chronically elevated cortisol levels can have several adverse effects. Most commonly, glucocorticoids cause insulin resistance, hyperglycemia, hyperlipidemia, and central obesity, increasing the risk for hypertension and cardiovascular disease. Chronically elevated glucocorticoids also lead to immunosuppression, and thus an increased risk of infection and even cancer. They also produce feelings of stress and depression and can therefore lead to neurological disorders.

Van Rossum et al. reviews the cause of elevated cortisol in obesity. Factors that are known to stimulate endogenous cortisol production include alcohol intake, chronic stress, sleep deprivation, pain, and inflammation. In addition to increased endogenous production, altered metabolism of cortisol may underlie increased cortisol levels in obesity. This could be due to changes in cortisol metabolizing enzymes (11-β-hydroxysteroid dehydrogenases [11-βHSD], 5α and 5β-reductases, CYP3A4), bile acids, or severe steatosis of the liver as the major site of cortisol clearance (Elisabeth F.C. van Rossum, Obesity and cortisol: New perspectives on an old theme, Obesity Volume 25, Issue 3 p. 500-501, February 23, 2017).

Wester et al. suggests that a higher long-term cortisol exposure in obese patients may contribute to cardiovascular disease risk (Wester et al., Long-term cortisol levels measured in scalp hair of obese patients, Obesity (Silver Spring). 2014 Sep;22(9):1956-8, Epub 2014 May 23.).

Jachson et al. discloses that chronic exposure to elevated cortisol concentrations, assessed in hair, is associated with markers of adiposity and with the persistence of obesity over time (Jackson et al., Hair cortisol and adiposity in a population-based sample of 2,527 men and women aged 54 to 87 years, Obesity (Silver Spring) 2017 Mar;25(3):539-544.).

Noppe et al. discloses that long-term cortisol concentrations are strongly associated with an increased risk of childhood obesity and adverse body-fat distribution (Noppe et al., Long-term glucocorticoid concentrations as a risk factor for childhood obesity and adverse body-fat distribution, Int J Obes (Lond). 2016 Oct;40(10):1503-1509. Epub 2016 Jun 24.).

Drugs to reduce the overproduction or function of cortisol are being developed primarily to treat Cushing's syndrome. In contrast to obesity, cortisol levels are extremely high in Cushing's syndrome. In non-pharmacologic Cushing's syndrome, there is usually a tumor in the pituitary or adrenal cortex, resulting in very high cortisol levels. First-line therapy is surgical removal of the pituitary tumor or uni- or bilateral removal of the adrenal glands with lifelong hormone replacement therapy. Drugs that inhibit steroidogenesis in the adrenal glands (osilodrostat, levoketoconazole, and ATR-101) are being tested in clinical trials but may have severe side effects. Mifepristone, an inhibitor of cortisol function, is used to treat type 2 diabetes mellitus or glucose intolerance in patients with Cushing's syndrome.

Rijjal et al (Vascepa protects against high-fat diet-induced glucose intolerance, insulin resistance, and impaired b-cell function, iScience 24, 102909, August 20, 2021) discloses that vascepa (icosapent ethyl) was administered to obese mice caused by a high-fat diet. It was concluded that the effects are attributed to enhanced expression of the fatty acid oxidation gene by vascepa. However, Rijjal et al does not mention that vascepa inhibits the function or production of cortisol.

Despite efforts in the prior art to develop appropriate pharmacological treatments, there remains an urgent need for alternative or improved substances for treating obesity, in particular to reduce the cortisol levels of patients with obesity.

### SUMMARY OF THE INVENTION

In light of the prior art the technical problem underlying the present invention is to provide alternative and/or improved means for the treatment and/or prevention of obesity in a human subject.

This problem is solved by the features of the independent claims. Preferred embodiments of the present invention are provided by the dependent claims.

The invention therefore relates to an eicosapentaenoic acid (EPA) ester derivative for use in the prevention and/or treatment of obesity in a human subject.

The invention therefore relates to an FADS2 inhibitor, such as an eicosapentaenoic acid (EPA) ester derivative or SC-26196, for use in the prevention and/or treatment of obesity in a human subject.

In another aspect, the invention relates to a method for treating obesity in a human subject, preferably central obesity, wherein the subject is treated with an EPA ester derivative. In embodiments, the subject has levels of cortisol, preferably cortisol levels in a hair sample or a blood sample, preferably a plasma sample, above a reference value and/or above an average cortisol level of healthy human subjects.

In a further aspect, the invention relates to a method for reducing cortisol levels in a human subject, wherein the subject is administered with an EPA ester derivative. In embodiments, the subject has levels of cortisol, preferably cortisol levels in a hair sample or a blood sample, preferably a plasma sample, above a reference value and/or above an average cortisol level of healthy human subjects.

In a further aspect, the invention relates to use of an EPA ester derivative for the manufacture of a medicament for the prevention and/or treatment of obesity. In embodiments, the subject has levels of cortisol, preferably cortisol levels in a hair sample or a blood sample, preferably a plasma sample, above a reference value and/or above an average cortisol level of healthy human subjects.

The invention further relates to a pharmaceutical composition comprising an EPA ester derivative with one or more pharmaceutical excipients for use in the prevention and/or treatment of obesity in a human subject.

The various aspects of the invention are unified by, benefit from, are based on and/or are linked by the common and surprising finding that glucocorticoid production was reduced by an EPA ester derivative. In particular, the effect of EPA can be attributed to the inhibition of fatty acid desaturase 2 (FADS2), which mediates the synthesis of arachidonic acid, thereby promoting glucocorticoid production.

In one aspect, the invention relates to an eicosapentaenoic acid (EPA) ester derivative for use in the prevention and/or treatment of obesity in a human subject.

Cortisol and the mineralcorticoid aldosterone are produced in the zona glomerulosa and zona fasciculata of the adrenal cortex, respectively. Aldosterone increases the blood volume and pressure, while cortisol mediates stress responses, including regulation of glucose and lipid metabolism (1-3). Excess production of aldosterone in primary hyperaldosteronism can cause resistant hypertension and hypokalemia (4). Hypercortisolismus leads to severe comorbidities, including central obesity, dyslipidemia and hypertension, collectively termed Cushing syndrome (5). Obesity is also associated with increased aldosterone and glucocorticoid production, which exacerbates central obesity, hyperlipidemia, hypertension and risk for cardiovascular disease (6-10).

Adrenocorticotropic hormone (ACTH), released by the pituitary, triggers cAMP signaling and induces glucocorticoid synthesis in the steroidogenic cells of the zona fasciculata. Corticosteroid synthesis requires the release of cholesterol from lipid droplets where it is stored in the form of cholesterol esters (CE), and its translocation into mitochondria via the steroidogenic acute regulatory (StAR) protein, which is the rate-limiting step of steroidogenesis. Once inside the mitochondria, cholesterol is used for steroidogenesis through a series of synthetic steps.

The present invention is based in part on the surprising and beneficial finding of the impact of mitochondrial membrane lipids on steroidogenesis in adrenocortical cells. Particularly, the inhibition of fatty acid desaturase 2 (FADS2), the rate-limiting enzyme of polyunsaturated fatty acid (PUFA) synthesis, which can be achieved by the compounds of the present invention, transforms the mitochondrial lipidome, inhibits cholesterol import and/or diminishes steroidogenesis.

Corticosteroids regulate vital processes, including stress responses, systemic metabolism and blood pressure. It was unexpected that corticosteroid synthesis is related to the polyunsaturated fatty acid (PUFA) content of mitochondrial phospholipids in adrenocortical cells. Inhibition of the rate-limiting enzyme of PUFA synthesis, fatty acid desaturase 2 (FADS2), leads to perturbations in the mitochondrial lipidome and diminishes steroidogenesis. Consistently, the adrenocortical mitochondria of Fads2-/- mice, fed a diet with low PUFA concentration, are structurally impaired and corticoid levels are decreased. On the contrary, FADS2 expression is elevated in the adrenal cortex of obese mice and plasma corticosterone is increased, which can be counteracted by dietary supplementation with an FADS2 inhibitor, such as SC- 26192, an EPA ester derivative, preferably icosapent ethyl, an eicosapentaenoic acid ethyl ester. Surprisingly, it has been found in humans that FADS2 expression is elevated in aldosterone-producing adenomas compared to non-active adenomas or non-tumorous adrenocortical tissue and correlates with expression of steroidogenic genes. These unexpected findings demonstrate that FADS2-mediated PUFA synthesis determines adrenocortical steroidogenesis in health and disease, and that EPA ester derivatives play a therapeutic role in modulating steroidogenesis, in particular cortisol production.

Surprisingly, FADS2 deficiency impairs mitochondrial structure in adrenocortical cells and reduces corticosterone and aldosterone production. Conversely, FADS2 expression is upregulated in the adrenal glands of obese mice and in aldosterone-producing adenomas compared to non-active adenomas (producing low amounts of aldosterone) and non-tumorous adrenocortical tissue of patients, while FADS2 inhibition reduces corticoid levels in obese mice. These findings demonstrate that FADS2 determines mitochondrial structure and function and highlights the crucial role of FADS2 in adrenocortical steroidogenesis. Therefore, the administration of eicosapentaenoic acid (EPA) ester derivative for the prevention and/or treatment of obesity in a human subject via regulating cortisol levels is a novel and unexpected finding that could not have been derived from the prior art.

Of note, reducing the function or production of cortisol levels in an obese subject by an EPA ester derivative is a technical effect clearly distinct form the mechanism described by Rijjal et al., in which the anti-obese effect is attributed to enhanced expression of fatty acid oxidation gene by vascepa.

Importantly, the novel technical effect underlying the present invention represents a novel therapeutic application of an EPA ester derivative. This surprising effect identifies a new clinical situation and enables a new therapeutic approach to treating obesity, namely by reducing the function and/or production of cortisol levels in the obese subject. Novel patient groups for treatment are identified for the inventive use of the EPA ester derivative, for example, obese subjects with above average cortisol levels. The novel clinical situation therefore exists through the underlying technical effect of the invention.

Subjects having obesity, in particular central obesity, are one patient group treatable by the invention. Especially, the subjects with above average levels of cortisol are also treatable by the invention. Subjects that do not respond to other anti-obesity therapies or other drugs for treating obesity may also be treatable by the invention. The invention may therefore be considered as a booster to the existing options for anti-obesity therapy or drugs currently available.

A novel clinical situation also arises through the mechanism underlying the invention, in that an EPA ester derivative reduces the function and production of cortisol through inhibiting FADS2. Icosapent ethyl (vascepa) is known for treating patients with cardiovascular disease, which is distinct from the patient group treatable by the invention. This new clinical situation therefore enables a novel use of an approved drug.

In some embodiments, the eicosapentaenoic acid (EPA) ester derivative is to be applied and/or administered in its free form, in other words free from any given polymeric structure encompassing EPA. The provision of solid forms of EPA, such as pharmaceutical salts or crystalline forms, are considered to fall under the non-polymeric free form of the substance.

In embodiments, the EPA ester derivative is for use in the prevention and/or treatment in central obesity. Central obesity is exacerbated by the increased aldosterone and glucocorticoid production. The surprising findings represent a novel and advantageous method for reducing the production of glucocorticoid production, thereby preventing and treating central obesity in a human subject.

In embodiments, the EPA ester derivative is for use in the prevention and/or treatment of peripheral obesity. In the peripheral obesity type, fat is accumulated around the hip and thigh areas.

In the context of the present invention, central obesity and peripheral obesity are not mutually exclusive but may overlap in some patients.

In embodiments, the obesity to be treated is associated with cortisol levels above the average levels of healthy human subjects. In embodiments, the subject has levels of cortisol above a reference value that may be determined by a skilled person. In embodiments, the subject has levels of cortisol above a normal range of healthy human subjects. Cortisol levels of the obese subject can be detected in blood, urine, saliva and/or hair.

In a preferred embodiment, the subject has levels of cortisol in a blood sample, preferably a plasma sample, above a reference value and/or above an average cortisol level, and/or out of the normal range of healthy human subjects. In another preferred embodiment, the subject has levels of cortisol in hair above a reference value and/or above an average cortisol level, and/or out of the normal range of healthy human subjects. For example, obese patients had higher hair cortisol levels than overweight and normal weight subjects (respectively 30.8 vs 8.5 and 8.4 pg/mg hair). No significant difference in hair cortisol levels was found between normal weight and overweight subjects.

Relevant Cortisol ranges may be determined by a skilled person without undue effort according to reports in the relevant literature. Determining an appropriate cut-off or reference value to identify patients suitable for the inventive treatment is within the routine skill of a relevant professional.

In embodiments, obese patients have hair cortisol levels equal to or above 9, 10, 11, 12, 13, 14, 15, 16, 17, 18, 19, 20, 21, 22, 23, 24, 25, 26, 27, 28, 29, 30, 31, or 31 pg/mg hair. In embodiments, obese patients have hair cortisol levels between 9-50 pg/mg hair, preferably 10-50 pg/mg hair, more preferably 15-40 pg/mg hair. In other embodiments, a threshold value can be used to compare determined hair cortisol levels. In embodiments, a threshold value may be employed that lies in a range according to 9-50 pg/mg, preferably 10-50 pg/mg, more preferably 15-40 pg/mg.

Advantageously, the measurement of hair cortisol appears to differentiate obese vs non-obese subjects with a greater accuracy compared to the use of plasma samples, since it reflects the cumulative cortisol levels over a period of several months.

In other embodiments, obese patients have urine cortisol levels equal to or above 50 µg/24h, or equal to or above 60 µg/24h, 70, 80, 90, 100, 110, 120, 130, 140, or 150 µg/24h or more. In other embodiments, a threshold value can be used to compare determined urine cortisol levels. In embodiments, a threshold value may be employed that lies in a range according to 50 to 150 µ g/24h, preferably 80-140 µg/24h, more preferably 100-130 µg/24h. Threshold values or cutoff values may, in embodiments, correspond in essence to the average values in a healthy human population, or by identifying values in terciles, where patients selected for treatment may, in embodiments, be in the second or third terciles, thus identifying patients with elevated levels of cortisol in urine, in hair, or other suitable samples.

In other embodiments, cortisol levels in a blood sample from an obese subject to be treated are - at 6 a.m. to 8 a.m. - above 15 mcg/dL and/or - at 3 p.m. to 5 p.m. - above 5 mcg/dL. In embodiments, cortisol levels in a blood sample from an obese subject to be treated are - at 6 a.m. to 8 a.m. - above 20 mcg/dL and/or - at 3 p.m. to 5 p.m. - above 10 mcg/dL.

In embodiments, an above average level or beyond a normal range of cortisol is indicative of obesity and/or an increased risk of having or developing obesity.

Cortisol, the primary stress hormone, increases glucose in the blood stream, enhances the brain's use of glucose and increases the availability of substances that repair tissues. Cortisol also curbs functions that would be non-essential or harmful in a fight-or-flight situation. It alters immune system responses and suppresses the digestive system, the reproductive system and growth processes. Elevated cortisol levels are therefore associated with obesity, although not all subjects with any given level of obesity exhibit elevated cortisol levels.

Advantageously, EPA ester derivatives could reduce the production, overproduction and/or function of cortisol, so that cortisol secretion and active cortisol in circulatory concentrations are decreased. The treatment according to the present invention represents an effective way to treat obese subjects with elevated levels of cortisol.

In embodiments, the subject suffers from depression and/or stress. For example, plasma cortisol levels have been measured in patients with a diagnosis of depressive disorder. Advantageously, by treating with EPA ester derivative the cortisol levels decrease and as such, in embodiments, the EPA ester derivative is suitable for use in anti-stress therapy, in patients with or without obesity. Surprisingly, eicosapentaenoic acid at a daily dose of 1 g has been shown to decrease cortisol levels in patients with depression, although no link with cortisol and obesity has been described in the art. Icosapent ethyl could therefore be used in an appropriate anti-stress therapy.

In embodiments, the EPA ester derivative is eicosapentaenoic acid ethyl ester (icosapent ethyl).

Icosapent-ethyl (also known under the tradename Vascepa^{®}) is a highly purified and stable ethyl ester of eicosapentaenoic acid, which is known for use in patients with elevated triglyceride levels to reduce cardiovascular risk (daily dose of fish extracts, mainly from essential cardioprotective effects 4 g). Rijjal et al further discloses that vascepa was administered to obese mice caused by a high-fat diet. However, obesity in mice and human subjects are determined by different phenotypes. Obesity in mice is determined by body fat mass, whereas human obesity is determined by BMI. Especially, due to the limitation of body form, it is difficult to establish a mouse model for central obesity.

To the knowledge of the inventors, the use of icosapent-ethyl has not been previously proposed for preventing or treating obesity, especially central obesity in a human subject. To the knowledge of the inventors, icosapent-ethyl has not been reported to reduce cortisol levels in obese human subjects.

Surprisingly, icosapent-ethyl has been shown to lower glucocorticoid levels in mice fed a high-fat diet for 10 weeks followed by 10 weeks of the high-fat diet with or without icosapent-ethyl (1.125 g/kg diet weight, 180 mg/kg body weight). Furthermore, in vitro studies indicate that eicosapentaenoic acid strongly reduces glucocorticoid production in human and primary murine adrenocortical cells. This effect of eicosapentaenoic acid can be attributed to the inhibition of fatty acid desaturase 2 (FADS2). FADS2 mediates the synthesis of arachidonic acid, thereby promoting glucocorticoid production. Accordingly, cortisol levels are reduced in obese patients via the inventive treatment. These surprising findings enable the use of icosapent-ethyl for treating a novel patient group, i.e. obese patients, through lowering cortisol levels.

In embodiments, glucocorticoid levels, such as cortisol levels, are reduced in the patient upon treatment.

Advantageously, the EPA ester derivative, for example, icosapent ethyl, lowers glucocorticoid levels in subjects with a high-fat diet. It has also been demonstrated in an in vitro study that icosapent ethyl lowers glucocorticoid, such as cortisol, in human and primary murine adrenocortical cells.

In embodiments, the EPA ester derivative inhibits the expression of fatty acid desaturase 2 (FADS2) in certain tissues, such as in fibroblasts, and the liver, preferably in the adrenal cortex in the patient upon treatment.

In embodiments, transcription of the fatty acid desaturase 2 (FADS2) gene is altered in the adrenal cortex upon treatment. The transcription of the FADS2 can be altered, preferably suppressed, by an EPA ester derivative, preferably icosapent ethyl.

In embodiments, suitable patients are identified by levels of activity and/or transcription of fatty acid desaturase 2 (FADS2). In embodiments, the subject has levels of FADS2 transcription above a reference value that may be determined by a skilled person. In embodiments, the subject has protein levels or activity levels of FADS2 above a normal range of healthy human subjects. FADS2 transcription levels in an obese subject can be detected in various samples, such as blood, urine, saliva and/or hair, or any given sample in which transcriptional changes in the adrenal cortex may be determined. Relevant FADS2 transcription levels may be determined by a skilled person without undue effort. Determining an appropriate cut-off or reference value to identify patients suitable for the inventive treatment is within the routine skill of a relevant professional.

Surprisingly, the lipidomic alternations caused by icosapent ethyl treatment were accompanied by transcriptional changes in the adrenal cortex, which has been demonstrated by RNA-seq experiments, in which 266 genes are up-regulated and 162 genes are down-regulated after the corresponding treatment. In addition, GSEA analysis of RNA sequencing data showed negative enrichment of genes related to fatty acid metabolism, cholesterol homeostasis and steroid biosynthesis in the adrenal cortex of the EPA ester derivative treated subject. More surprisingly, genes related to mitochondrial envelope formation, respiratory electron transport, oxidative phosphorylation and TCA cycle were negatively enriched in the adrenal cortex of the treated subject. These surprising findings enable an effective anti-obesity therapy with the EPA ester derivative and provide a novel technical effect, thus enabling more specific selection of patients for the appropriate treatment based on cortisol levels and/or other transcriptional changes in the adrenal cortex.

In embodiments, the cortisol and/or aldosterone levels are reduced in the patient upon treatment, without altering the adrenocorticotropic hormone (ACTH) plasma levels. This demonstrates that treatment with the EPA ester derivative is associated with lipidomic and transcriptional reprogramming of the adrenal cortex, suggesting an intra-adrenal regulation of corticosteroid production.

In embodiments, the concentration of polyunsaturated fatty acids (PUFA) (preferably omega-3 fatty acids) is increased in tissues of the patient upon treatment.

It has been surprisingly found that the concentration of PUFA is increased in the tissues of the patient upon treatment of the EPA ester derivative. In particular, the ratio of omega-3 vs omega-6 is increased, which advantageously reduces the risk of chronic diseases.

In embodiments, the levels of pro-inflammatory lipid mediators are decreased in tissues of the patient upon treatment. Advantageously, the obese subject can benefit from decreased levels of pro-inflammatory lipid mediators, providing additional benefit to the patient associated with reducing cortisol levels.

In embodiments, the patient has or is undergoing a combined (preferably simultaneous or sequential) treatment with one or more other active agents for treating obesity and/or other medical treatments for obesity, such as surgery.

In further aspect, the invention relates to an FADS2 inhibitor, such as SC-26196, for use in the prevention and/or treatment of obesity in a human subject.

Surprisingly, FADS2 inhibition reduces corticosteroid levels in obese mice. It has been shown that corticosterone and 11-dehydrocorticosterone levels were significantly reduced in mice which were fed with HFD for 14 weeks and received a FADS2 inhibitor the last 6 weeks of the feeding period.

In a further aspect and embodiments, a pharmaceutical composition comprising an EPA ester derivative, with one or more pharmaceutical excipients, for the intended medical use is described herein.

The administration of the compositions contemplated herein may be carried out in any convenient manner, including by injection, ingestion, transfusion, topical application or aerosol inhalation. In a preferred embodiment, the compound is administered by ingestion. In some embodiments, "systemic administration" is used, and refers to the administration of a composition that results in broad biodistribution drug or the derivatives thereof within an organism. Systemic administration means exposing a therapeutic amount of an agent to preferred parts of the body. Systemic administration of the composition may be accomplished by any means known in the art, e.g., intravenously, subcutaneously, intraperitoneal. Modes of administration are also considered other than enteral and topical administration, for example by injection, including, without limitation, intravascular, intravenous, intramuscular, intraarterial, intrathecal, intracapsular, intraorbital, intratumoral, intracardiac, intradermal, intraperitoneal, transtracheal, subcutaneous, subcuticular, intraarticular, subcapsular, subarachnoid, intraspinal and intrasternal injection and infusion.

The embodiments describing the EPA ester derivative of the invention may be used to describe other aspects of the invention, including the pharmaceutical composition comprising the EPA ester derivative, and vice versa. Analogously, the EPA ester derivative of the invention may be used to describe the medical use thereof and vice versa. Each embodiment described herein may therefore be used to describe any other embodiment or aspect, as would be understood by a skilled person. The invention is unified by the novel and beneficial use of the EPA ester derivative as described herein, and thus the relevant features described one aspect may be used to describe any given aspect of the invention, in a manner in conformity with the understanding of a skilled person.

### DETAILED DESCRIPTION OF THE INVENTION

All cited documents of the patent and non-patent literature are hereby incorporated by reference in their entirety.

The term "eicosapentaenoic acid (EPA)" as used herein refers to an omega-3 fatty acid, namely a carboxylic acid with a 20-carbon chain and five cis double bonds, as established in the art (also known under CAS Number 10417-94-4). EPA is a polyunsaturated fatty acid (PUFA) that acts as a precursor for prostaglandin-3, which inhibits platelet aggregation, thromboxane-3, and leukotriene-5 eicosanoids. EPA is both a precursor and the hydrolytic breakdown product of eicosapentaenoyl ethanolamide (EPEA: C22H35NO2; 20:5,n-3).

Eicosapentaenoic acid (EPA) refers inclusively to (5Z,8Z,1 IZ,14Z,17Z)-eicosa- 5,8,11,14,17-pentenoic acid or derivatives thereof, including alkyl esters, such as, for example, the ethyl ester, or other esters, such as, for example, the monoglyceride, diglyceride or triglyceride ester.

In embodiments, eicosapentaenoic acid refers to an eicosapentaenoic acid ester derivative.

In embodiments, an ester derivative of eicosapentaenoic acid is icosapent ethyl. In embodiments, icosapent ethyl is a highly purified and stable ethyl ester of eicosapentaenoic acid, which is known for using in patients with elevated triglyceride levels to reduce cardiovascular risk (daily dose of fish extracts, mainly from essential cardioprotective effects 4 g). Icosapent ethyl is also known as ethyl eicosapentaenoic acid (E-EPA) or CAS Number 86227-47-6.

As used herein, the term "ester" refers to the replacement of the hydrogen in the carboxylic acid group of a polyunsaturated fatty acid molecule with another substituent. Typical esters are known to those in the art, a discussion of which is provided by Higuchi, T. et al., Pro-drugs as Novel Delivery Systems, Vol. 14, A.C.S. Symposium Series, Bioreversible Carriers in Drug Design, Ed. Edward B. Roche, Amer. Pharma. Assoc., Pergamon Press (1987), and Protective Groups in Organic Chemistry, McOmie ed., Plenum Press, New York (1973), each of which is incorporated herein by reference in the entirety. Examples of common esters include methyl, ethyl, trichloroethyl, propyl, butyl, pentyl, tert-butyl, benzyl, nitrobenzyl, methoxybenzyl, benzhydryl, monoglyceride, diglyceride, triglyceride.

As used herein, a "fatty acid" is a carboxylic acid with a long aliphatic chain, which is either saturated or unsaturated. Most naturally occurring fatty acids have an unbranched chain of an even number of carbon atoms, from 4 to 28. Fatty acids are mainly found as components of three main classes of esters: triglycerides, phospholipids, and cholesteryl esters. In any of these forms, fatty acids are both important dietary sources of fuel for animals and they are important structural components for cells. Fatty acid esters (FAEs) are a type of ester that result from the combination of a fatty acid with an alcohol. When the alcohol component is glycerol, the fatty acid esters produced can be monoglycerides, diglycerides, or triglycerides. Dietary fats are typically triglycerides.

The term "Polyunsaturated fatty acids (PUFA)" refers to fatty acids that contain more than one double bond in their backbone. This class includes many important compounds, such as essential fatty acids and those that give drying oils their characteristic property. Polyunsaturated fats are fats in which the constituent hydrocarbon chain possesses two or more carbon-carbon double bonds. Polyunsaturated fat can be found mostly in nuts, seeds, fish, seed oils, and oysters. "Unsaturated" refers to the fact that the molecules contain less than the maximum amount of hydrogen (if there were no double bonds). These materials exist as cis or trans isomers depending on the geometry of the double bond. Polyunsaturated fatty acids include, but are not limited to, omega-3 fatty acids, omega-6 fatty acids, omega-9 fatty acids, pinolenic acid and sciadonic acid.

As used herein, "Omega-3 fatty acids", also called Omega-3 oils, ω-3 fatty acids or n-3 fatty acids, are polyunsaturated fatty acids (PUFAs) characterized by the presence of a double bond three atoms away from the terminal methyl group in their chemical structure. They are widely distributed in nature, being important constituents of animal lipid metabolism, and they play an important role in the human diet and in human physiology. Three types of omega-3 fatty acids involved in human physiology are α-linolenic acid (ALA), found in plant oils, and eicosapentaenoic acid (EPA) and docosahexaenoic acid (DHA), both commonly found in marine oils, such as codliver oil.

As used herein, the term "triglyceride" refers to a fatty acid chain, such as EPA, covalently bonded to a glycerol molecule through an ester linkage, wherein the glycerol molecule is further bonded to two additional fatty acid chains, either or both of which may or may not be EPA, through two additional ester linkages.

The term "obesity" as used herein refers to abnormal or excessive fat accumulation in a subject. In embodiments, obesity shall also mean overweight. In embodiments, the obesity is characterized as a pathological or medical condition. Obesity can be identified using the body mass index (BMI). In embodiments, obesity refers to a body mass index (BMI) over 25, 26, 27, 28, 29, 30, 31, 32, 33 and 34. In preferred embodiments, obesity refers to a body mass index over 30.

In embodiments, obesity can be assessed by anthropomorphic measurements (e.g., waist circumference, waist-height ratio, waist-hip ratio, etc.), biological measurements (hyper-triglyceridemic waist, metabolites, genomic markers, etc.), and imaging (e.g., CT, MRI, DXA, etc.).

In embodiments, obesity can be assessed by a number of metabolites that are known to be associated with BMI and obesity. These include branched chain amino acids (leucine, isoleucine, valine), aromatic amino acids (tyrosine, tryptophan), uric acid, phospholipids, glucose, mannose, asparagine, glycerol, and glycerophosphocholines.

The term "central obesity", also called "abdominal obesity", as used herein refers generally to obesity with an excess accumulation of fat in the abdominal area. In embodiments, central obesity is due to excess visceral fat. In embodiments, central obesity is defined as the condition wherein the waist circumference is >40 inches or 102 cm in men, and is >35 inches or 94 cm in women. However, the definition varies due to gender, age and race. For example, with regard to a Japanese ethnicity or Japanese patients abdominal obesity may be defined as waist circumference ≥85 cm in men and ≥90 cm in women.

The term "visceral obesity" is defined as a condition wherein a waist-to-hip ratio of greater than or equal to 1.0 in men and 0.8 in women is measured. It defines the risk for insulin resistance and the development of pre-diabetes.

The term "peripheral obesity" as used herein refers to a condition where fat is accumulated around the hip and thigh areas.

In embodiments, a subject diagnosed with central obesity could have peripheral obesity and visceral obesity. In embodiments, a subject diagnosed with peripheral obesity could have central obesity and visceral obesity. In embodiments, a subject diagnosed with visceral obesity could have central obesity and peripheral obesity.

The term "cortisol" refers to a well-known glucocorticoid hormone that adrenal glands produce and release. Cortisol may be identified by (1*R*,3a*S*,3b*S*,9a*R*,9b*S*,11a*S*)-1,10-Dihydroxy-1-(hydroxyacetyl)-9a,11a-dimethyl-1,2,3,3a,3b,4,5,8,9,9a,9b,10,11,11a-tetradecahydro-7*H-*cyclopenta[a]phenanthen-7-one. Cortisol regulates the body's stress response, helps control the body's use of fats, protein and carbohydrates or metabolism, suppresses inflammation, and regulates blood pressure and blood sugar. Glucocorticoids are a type of steroid hormone which suppress inflammation in all of bodily tissues and control metabolism in muscles, fat, liver and bones. Cortisol can be determined in a sample of blood, urine, saliva or hair using established methods, depending on the protocol of the test provider. Such tests are available as Lykon, Cerascreen and Verisana.

The term "average cortisol level of a healthy human population" shall mean the normal cortisol level with respect to a non-obese population. In embodiments, a healthy human population refers to a non-obese population that does not exhibit any other medical conditions. In embodiments, a population with other non-obesity related diseases could be understood as a healthy human population. The cortisol level can be measured, for example, in hair, blood, preferably in plasma, urine and saliva. The average cortisol level of healthy human subject is not limited to a specific value and depends on a number of factors, such as gender, age group or race. In embodiments, the average cortisol level of a healthy human population is given for certain race, certain area, with respect to gender and age.

In embodiments, any by way of example, in the USA the normal ranges at around 6 a.m. to 8 a.m. are 10-20 mcg/dL and around 4 p.m. 3-10 mcg/dL. In embodiments, cortisol levels in an obese subject to be treated are - at 6 a.m. to 8 a.m. - above 15 mcg/dL and/or - at 3 p.m. to 5 p.m. - above 5 mcg/dL. In embodiments, cortisol levels in an obese subject to be treated are - at 6 a.m. to 8 a.m. - above 20 mcg/dL and/or - at 3 p.m. to 5 p.m. - above 10 mcg/dL.

In other embodiments, normal values for a blood sample taken at 8 in the morning are 5-25 mcg/dL or 140-690 nmol/L. Normal values depend on the time of day and the clinical context. Normal ranges may vary slightly among different laboratories or testing methods.

Cortisol production and secretion are typically increased in obesity (Brix et al, Obes Facts (2021) 14 (5): 510-519; Dunkelman et al, The Journal of Clinical Endocrinology & Metabolism, Volume 24, Issue 9, 1 September 1964, 832-841; Purnell et al, The Journal of Clinical Endocrinology & Metabolism, Volume 89, Issue 1, 1 January 2004, 281-287) and lead to enhanced urinary free cortisol levels, as also shown in women with abdominal body fat distribution (Marin et al, Metabolism, Volume 41, 8, 882-886, August 1992; Pasquali et al, The Journal of Clinical Endocrinology & Metabolism, Volume 77, Issue 2, 1 August 1993, 341-346).

For example, Marin et al show that urinary cortisol output is typically increased in women with an elevated waist to hip circumference ratio (WHR) and, in particular, in women with a large abdominal sagittal diameter, indicating visceral fat accumulation.

By way of further example, Brix et al describe that in 1,249 nondiabetic patients (79.8% females, mean BMI 44.9 ± 6.5 kg/m2, mean age 38 ± 11 years), cortisol excretion was assessed on 2 consecutive days (24 h urine specimens), whereby patients were divided into 3 terciles based on cortisol levels (urinary cortisol ≤51.6, >51.6 and <117.6, and ≥117.6 µg/24 h, respectively). Patients in the highest tercile were younger (p = 0.003) and typically more obese (BMI: p = 0.040), among other characteristics. Increased urinary cortisol excretion in obesity is regarded as a consequence of overactivity of the hypothalamic-pituitary-adrenal (HPA) axis. Nevertheless, obese subjects show varying cortisol levels and patients in the present invention may in embodiments be elected based on elevated cortisol levels.

As known to a skilled person, determining cortisol levels in a subject and comparing determined levels to reference values or other established levels, for example in a healthy population, is established in the art and requires no undue effort. For example, serum cortisol assays can measure total cortisol. Automated immunoassays are also used to measure cortisol. Liquid chromatography - tandem mass spectrometry (LC-MS/MS) can also measure cortisol and may offer improved specificity and sensitivity. Urine free cortisol is often used to screen for Cushing's syndrome. Unbound cortisol is excreted unchanged in the urine and 24-h urine free cortisol correlates well with mean serum-free cortisol in conditions of cortisol excess. Urine free cortisol is measured predominantly by immunoassay or LC-MS/MS. Salivary cortisol also reflects changes in unbound serum cortisol and offers a reliable alternative to measuring free cortisol in serum.

Cortisol tests have been established and are well known in the art to detect Cushing syndrome or Addison's disease. Such tests are also known in the context of screening for other diseases that affect your pituitary and adrenal glands. Blood Test: Often, a cortisol blood test is done twice in the same day -- once in the morning, and again later in the afternoon, around 4 p.m. Saliva Test: Studies show that cortisol saliva tests are about 90% accurate in diagnosing Cushing syndrome. Saliva tests are typically conducted at night, as cortisol levels tend to be lowest between 11 p.m. and midnight. Urine Test: Urine tests typically assess free cortisol. This means the cortisol isn't bound to a protein as it is in blood tests. Urine tests typically require a 24-hour sample, in which urine is collected over the course of a full day.

Hair test: Scalp hair cortisol is also an established technique, and an emerging measure of cortisol levels and of cumulative hypothalamic-pituitary-adrenal (HPA) activity, without the limitations of other biological specimens (saliva, urine, blood) used to assess cortisol. Hair cortisol in the most proximal 3cm of hair may be employed. Cortisol obtained from scalp hair originates from the vascular supply which nourishes the hair shaft follicular cells. The source of cortisol within the hair shaft is from circulation to the medullary region in the core of the hair. Sources of cortisol on the surface of the hair include both sweat as well as sebaceous glands which are most likely eliminated by the washing steps prior to grinding the hair for extraction. It is generally thought that the cortisol measured using the standard wash, grind, and extraction method most likely reflects free cortisol and not the bound steroid.

Cortisol production and secretion are typically increased in obesity (Dunkelman et al, The Journal of Clinical Endocrinology & Metabolism, Volume 24, Issue 9, 1 September 1964, 832-841; Purnell et al, The Journal of Clinical Endocrinology & Metabolism, Volume 89, Issue 1, 1 January 2004, 281-287) and lead to enhanced urinary free cortisol levels, as also shown in women with abdominal body fat distribution (Marin et al, Metabolism, Volume 41, 8, 882-886, August 1992; Pasquali et al, The Journal of Clinical Endocrinology & Metabolism, Volume 77, Issue 2, 1 August 1993, 341-346). Increased urinary cortisol excretion in obesity is regarded as a consequence of overactivity of the hypothalamic-pituitary-adrenal (HPA) axis. Nevertheless, obese subjects show varying cortisol levels and patients in the present invention may be elected based on cortisol levels, if necessary.

The term "FADS2" refers to fatty acid desaturase 2 which is encoded by the FADS2 gene. Fatty acid desaturase 2 is a member of the fatty acid desaturase (FADS) gene family. Desaturase enzymes cause desaturation of fatty acids through the introduction of double bonds between defined carbons of the fatty acyl chain. EPA was previously shown to downregulate FADS2 expression in THP-1 cells, 3T3-L1 adipocytes and HepG2 hepatocytes. In embodiments, EPA treatment downregulates the FADS2 expression in adrenocortical cells and diminishes forskolin-stimulated cortisol production.

The term "FADS2 inhibitor" shall mean a substance, compound, molecule, mixture or system capable of interfering with the activity and/or expression or other functional property of FADS2. In one embodiment, an FADS2 inhibitor modulates the role of FADS2 negatively, for example, an eicosapentaenoic acid (EPA) ester derivative, such as icosapent ethyl. In one embodiment, an FADS2 inhibitor negatively modulates the function FADS2. In one embodiment, an FADS2 inhibitor disrupts the interaction between it and its counterpart. In one embodiment, a FADS2 inhibitor negatively regulates translation of the FADS2 mRNA. In embodiments, a FADS2 inhibitor functions on the post-translational level, for example by accelerating or inducing FADS2 protein degradation. In another embodiment, an FADS2 inhibitor functions at an RNA level and reduces the mRNA levels of FADS2, such as a FADS2-specific siRNA or shRNA, or other molecule suitable for reducing transcriptions of the FADS2-encoding gene. In some embodiments, an FADS2 inhibitor functions at a DNA level, which leads to knock-down or knock-out of FADS2, such as via CRISPR-Cas9.

In embodiments, an FADS2 inhibitor can be a compound, a modulator or a ligand or a small molecule inhibitor or antagonist or an antibody binding to FADS2 or an FADS2 siRNA, FADS2 shRNA, or any combinations thereof.

In embodiments, an FADS2 inhibitor can be an FADS2-suppressive oligonucleotide, such as an antisense oligonucleotide, a microRNA (miR), an shRNA, an siRNA, or a guide-RNA in conjunction with an RNA-guided endonuclease, or a nucleic acid molecule suitable for expression of a FADS2-suppressive oligonucleotide, such as an expression plasmid or viral vector encoding an FADS2-suppressive oligonucleotide. In embodiments, the FADS2 suppressor can be a system including several components, such as for example a guide RNA in conjunction with an RNA-guided endonuclease, for example Cas9, which may function for example by generating a functional knock-out of the FADS2 gene in the genomic DNA of a cell by means of the CRISPR/Cas9 system. In such a context, an FADS2 inhibitor system can comprise several components that act together to generate a functional inhibition, for example a functional gene knock-out, of FADS2. The skilled person in view of the present disclosure and the common general knowledge is aware of suitable system components that may lead to functional inhibition of FADS2 and that can be used as an FADS2 inhibitor in the sense of the invention.

In embodiments, a FADS2 inhibitor can be a compound, such as a biological or chemical molecule, such as a small molecule, a protein, a nucleic acid, chemicals, inorganic molecules, organic molecules, cDNA encoding a protein, a secreted protein, a large molecule, an antibody, a morpholino, a triple helix molecule, a peptide, siRNA, shRNA, miRNA, antisense RNA, ribozyme or any other compound or combination of compounds that can be envisioned to be used as a functional inhibitor of FADS2 in the treatment of a subject.

Examples of FADS2-inhibitors are, by way of example, conjugated linoleic acid (9Z,1 1E) (CAS: 2540-56-9), CP 24,879 (hydrochloride) (CAS: 10141-51-2), and SC 26196 (CAS: 218136-59-5). Other examples of FADS2-inhibitors are eicosapentaenoic acid (EPA), and ester derivatives of eicosapentaenoic acid, for example icosapent ethyl.

The term "adrenal cortex" as used herein shall mean the outer part of the adrenal gland which produces androgen, corticosteroid and mineralcorticoid hormones.

The term "pro-inflammatory lipid mediator" refers to a metabolite that has a pro-inflammatory activity, i.e, an activity that promotes an inflammatory condition. Examples of inflammation converging lipid mediators include resolvin, protectin, lipoxin, and maresin.

The term "BMI" refers to body mass index and is a value derived from the mass (weight) and height of a human subject. BMI is defined as the body mass divided by the square of the body height, and is expressed in units of kg/m2, resulting from mass in kilograms and height in meters. BMI is a convenient assessment to categorize a person as underweight, normal weight, overweight, or obese based on tissue mass (muscle, fat, and bone) and height. Major adult BMI classifications are underweight (under 18.5 kg/m2), normal weight (18.5 to 24.9), overweight (25 to 29.9), and obese (30 or more). Variation may occur depending on age, gender or other factors.

The term "subject" includes both humans and animals, particularly mammals, and other organisms. The human subject may be a pediatric, adult, or a geriatric subject. The human subject may be of any gender. Preferred subjects are those with a BMI of at least 25, 26, 27, 28, 29, 30, 31, 32, 33 or 34.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In one embodiment, "treatment" refers to the treatment of obesity wherein the EPA ester derivative is administered to a human subject to reduce the production and/or function of cortisol.

The present invention also relates to a pharmaceutical composition comprising a compound described herein. The invention also relates to pharmaceutically acceptable salts of the compound described herein, in addition to enantiomers and/or tautomers of the compounds described.

The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" or "carrier substance" or "diluent" or "excipient" includes without limitation any and all solvents, adjuvant, dispersion media, glidant, sweetening agent, vehicles, coatings, diluents, antibacterial and antifungal agents, flavor enhancer, surfactant, solvent, emulsifier, wetting agent, dispersing agent, isotonic and absorption delaying agents, buffers, carrier solutions, suspensions, colloids, and the like, and which has been also approved by e.g. the United States Food and Drug Administration or European Medicines Agency as being acceptable for use in humans or domestic animals. The use of such media and agents for pharmaceutical active substances is well known in the art. Supplementary active ingredients can also be incorporated into the compositions.

A pharmaceutical composition as used herein can be an EPA ester derivative alone or a composition comprising an EPA ester derivative, in combination with one or more pharmaceutical excipients. The pharmaceutical composition as used herein is administered to a subject in an amount which is effective for treating the specific disease or disorder. As used herein "pharmaceutical excipient" shall refer to any given acceptable substance useful in formulating a composition for the drug substance. Examples include, but are not limited to, calcium phosphates, calcium carbonate, calcium sulfate, halites, metallic oxides, sugars, actual sugars, sugar alcohols, artificial sweeteners, modified starch, dried starch, converted starch, cellulose ethers, cellulose esters, CMC, croscarmellose sodium, microcrystalline cellulose, polyethylene glycol, propylene glycol, povidones, petrolatum, mineral waxes, mineral oils, acrylic polymers, fatty alcohols, mineral stearates, glycerin, lipids, other oleochemical excipients and proteins.

The pharmaceutical composition containing the active ingredient may be in a form suitable for oral use, for example, as tablets, chewing tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions. Tablets contain the active ingredient in admixture with non-toxic pharmaceutically acceptable excipients which are suitable for the manufacture of tablets. The tablets may be uncoated, or they may be coated by known techniques to delay disintegration and absorption in the gastrointestinal tract and thereby provide a sustained action over a longer period.

Simultaneous administration of one or more active agents shall mean administration of one or more agents at the same time. For example, in one embodiment, the inhibition of FADS2 can be achieved by an EPA ester derivative or other FADS2 inhibitors at the same time. In other embodiment, the EPA ester derivative can be administrated together with another active agent useful for a blood vessel disorder.

Sequential administration of one or more active agents shall mean administration of the therapeutic agents in a sequential manner. In one embodiment, each therapeutic agent is administered at a different time. In other embodiments, by administration of two or more therapeutic agents wherein at least two of the therapeutic agents are administered in a sequential manner which is a substantially simultaneous manner. Substantially simultaneous administration can be accomplished, for example, by administering to the subject a single dose having a fixed ratio of each therapeutic agent or in multiple, single doses for each of the therapeutic agents.

As used herein, the term "simultaneously" refers to administration of one or more agents at the same time. For example, in certain embodiments, different FADS2 inhibitors or EPA ester derivatives in combination with other active agents useful for treating obesity are administered at simultaneously. Simultaneously includes administration contemporaneously, that is during the same period of time. In certain embodiments, the one or more agents are administered simultaneously in the same hour, or simultaneously in the same day. Sequential or substantially simultaneous administration of each therapeutic agent can be affected by any appropriate route including, but not limited to, oral routes, intravenous routes, subcutaneous routes, intramuscular routes, direct absorption through mucous membrane tissues (e.g., nasal, mouth, vaginal, and rectal), and ocular routes (e.g., intravitreal, intraocular, etc.). The therapeutic agents can be administered by the same route or by different routes. For example, one component of a particular combination may be administered by intravenous injection while the other component(s) of the combination may be administered orally. The components may be administered in any therapeutically effective sequence.

In some embodiments, an effective amount of the EPA ester derivative is present in the composition. As used herein, an "effective amount" or "therapeutically effective amount" of a composition as described in some embodiments herein can be a quantity sufficient to achieve a desired therapeutic and/or prophylactic effect, for example, an amount which results in the prevention of, or a decrease in the symptoms associated with, a disease that is being treated.

### FIGURES

The following figures are presented to describe particular embodiments of the invention, without being limiting in scope.

### Brief description of the figures:

**Figure 1****:** Icosapent-ethyl and eicosapentaenoic acid inhibit glucocorticoid production.
**Figure 2****:** Icosapent-ethyl reduces cortisol production in the adrenal gland.
**Figure 3****:** FADS2 determines the mitochondrial lipidome and is required for steroidogenesis in adrenocortical cells.
**Figure 4****:** High-fat diet reprograms the adrenal lipidome and increases glucocorticoid production.
**Figure 5****:** Medulla - derived PUFA promote adrenocortical steroidogenesis.
**Figure 6****:** Dietary supplementation with icosapent ethyl reduces corticoid levels in obese mice.
**Figure 7****:** FADS2 deficiency perturbs adrenal gland function.
**Figure 8****:** FADS2 expression is increased in aldosterone-producing adenomas.
**Figure 9****:** FADS2 inhibition reduces corticosteroid levels in obese mice.

### Detailed description of the figures:

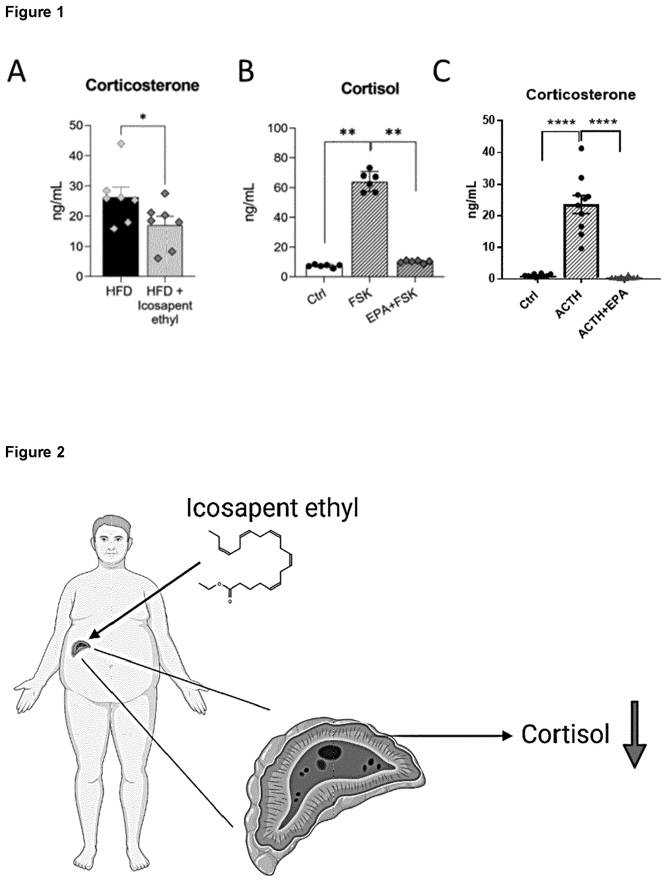

**Figure 1****:** Icosapent-ethyl and eicosapentaenoic acid inhibit glucocorticoid production. A. Mice were fed a high-fat diet for 10 weeks followed by the high-fat diet with or without icosapent-ethyl (1,125 g/kg diet weight, 180 mg/kg body weight) for 10 weeks. At the end of feeding, corticosterone levels were measured by liquid chromatography mass spectrometry (LC-MS/MS) in the blood plasma of the mice (n=7). B. Human adrenocortical cells (NCI-H295R) were treated with forskolin (FSK, 1 µM) and with or without eicosapentaenoic acid (EPA, 66 µM) for 48 hours, and cortisol was measured by LC-MS/MS in cell culture supernatant (n=6). C. Primary murine adrenocortical cells were treated with adrenocorticotropic hormone (ACTH, 10 ng/ml) and with or without eicosapentaenoic acid (EPA, 66 µM), and cortisosterone was measured by LC-MS/MS in cell culture supernatant (n=10). Results are shown as means ± standard errors, * p-value<0.05;** p-value<0.01; **** p-value<0.0001.

**Figure 2****:** Icosapent-ethyl reduces cortisol production in the adrenal gland. Cortisol is measured in patients before and after treatment with icosapent-ethyl.

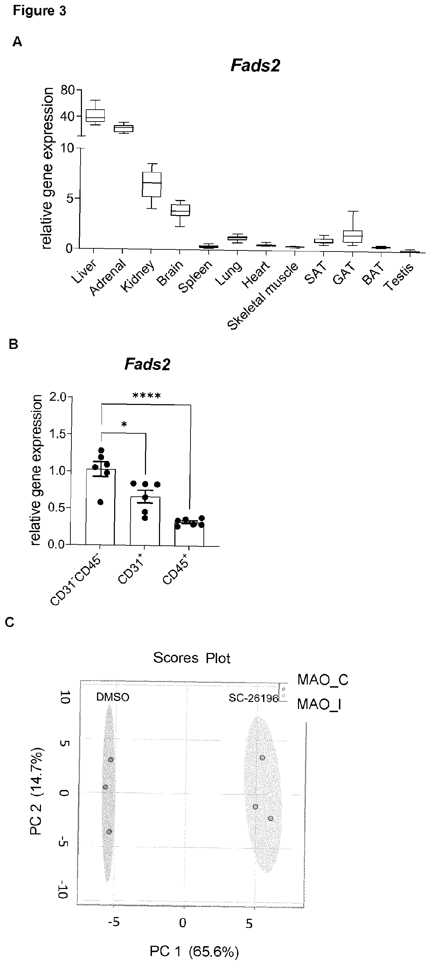

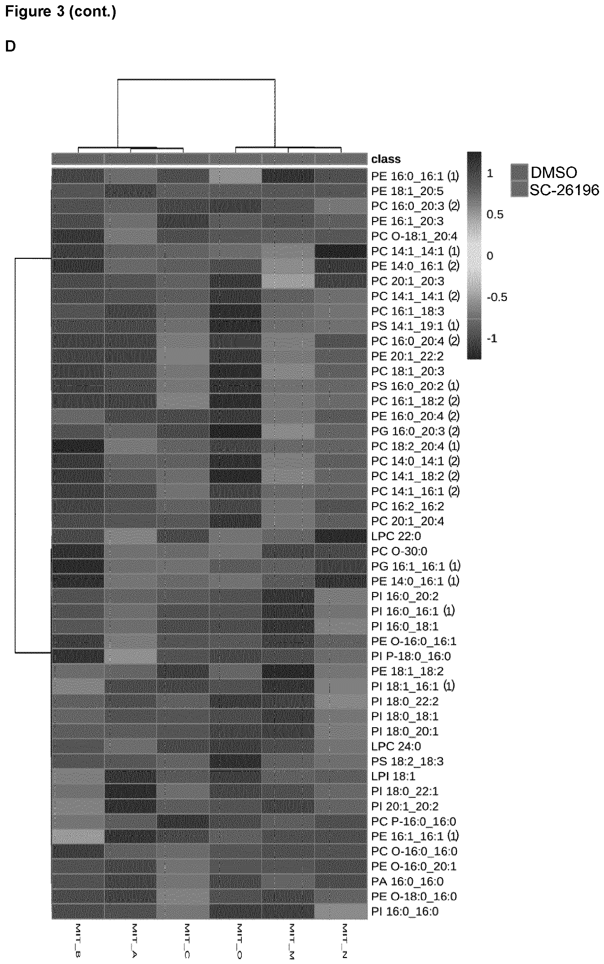

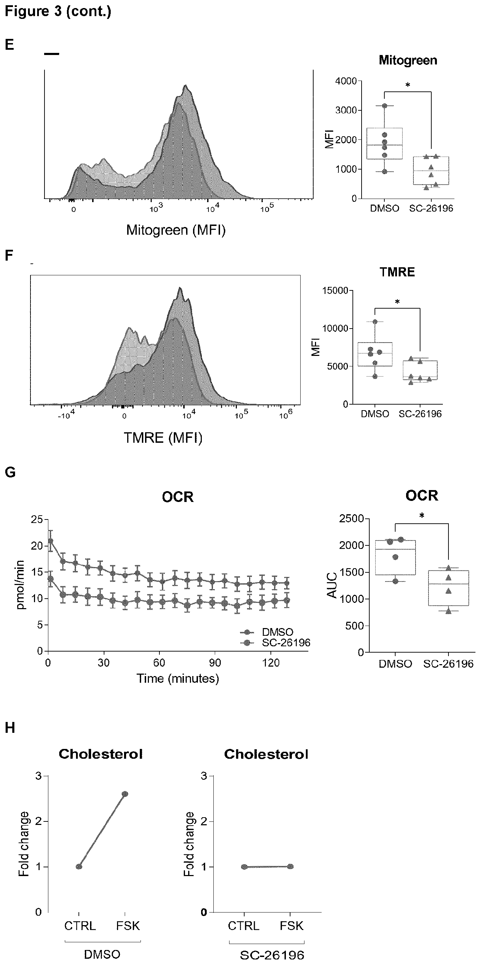

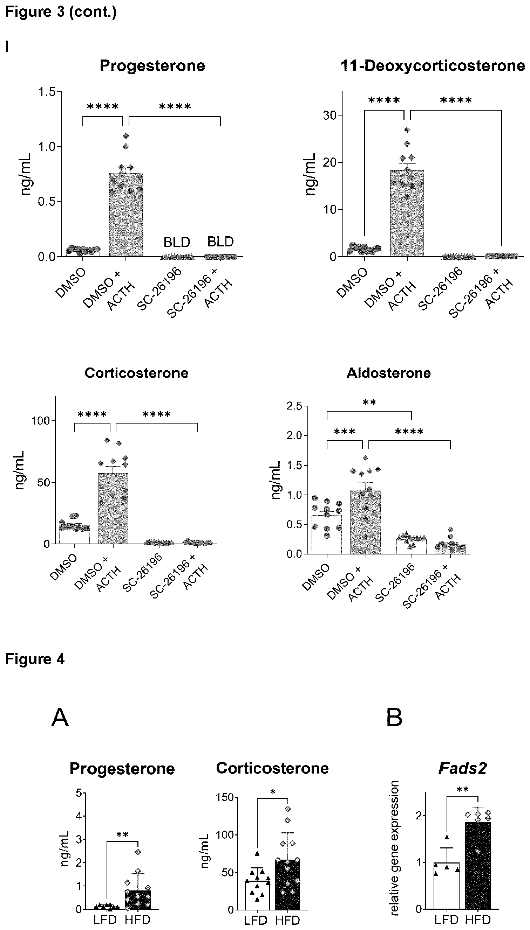

**Figure 3****:** A. Relative Fads2 expression in different tissues of 8-week-old WT C57BL6J mice determined by qPCR using *Tbp* as a housekeeping gene. Data are shown as mean 2^-ΔCt (n=8). BAT: brown adipose tissue, SAT: subcutaneous adipose tissue, GAT: gonadal adipose tissue. B. Relative Fads2 expression in CD31-CD45-, CD31⁺ and CD45⁺ cell populations of the mouse adrenal cortex. Expression of *Fads2* in CD31⁻CD45⁻ cells was set to 1. 18S was used as an internal control (n=6). C, D. PCA of the phospholipidome (C) and heatmap showing the differentially regulated lipid species (D) of mitochondrial fractions of NCI-H295R cells treated for 18 h with SC-26196 (10 µM) or equal amount of DMSO (n=3, one out of two experiments). E,F. Mitogreen (E) and TMRE (F) staining in primary adrenocortical cells treated for 18 h with SC-26196 (10 µM) or DMSO (n=6). G. OCR measurement of primary adrenocortical cells treated for 18 h with SC-26196 or DMSO (n=4). H. Cholesterol levels determined by LC-MS/MS in mitochondrial fractions of NCI-H295R cells treated for 18 h with SC-26196 or DMSO and stimulated or not with forskolin (FSK, 1 µM) for 30 min. One out of two experiments shown here. I. Progesterone, 11-deoxycorticosterone, corticosterone and aldosterone levels were determined by LC-MS/MS in supernatants of primary adrenal cell cultures treated for 18 h with SC-26196 or DMSO, and then stimulated or not for 1 h with ACTH (10 ng/mL) in presence of SC-26196 or DMSO. The cell culture medium was changed prior to ACTH treatment (n=12). Data in B and I are shown as mean ± SEM, * p-value<0.05;** p-value<0.01; **** p-value<0.0001

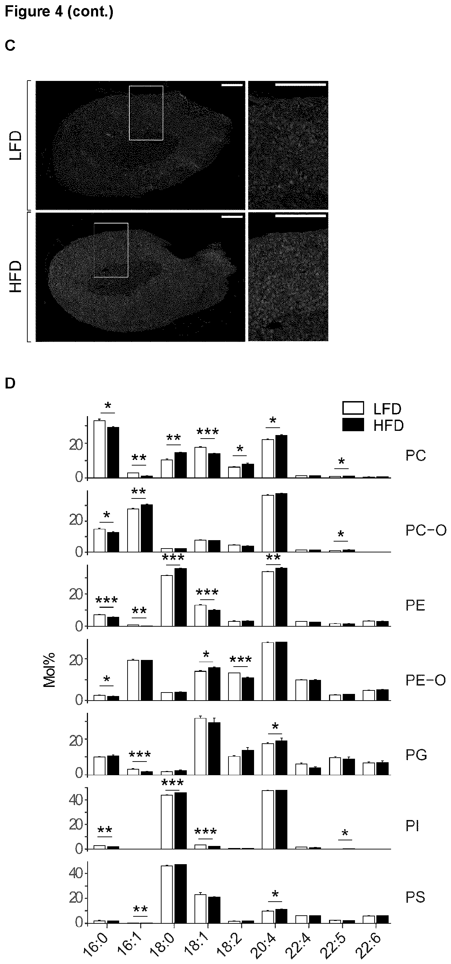

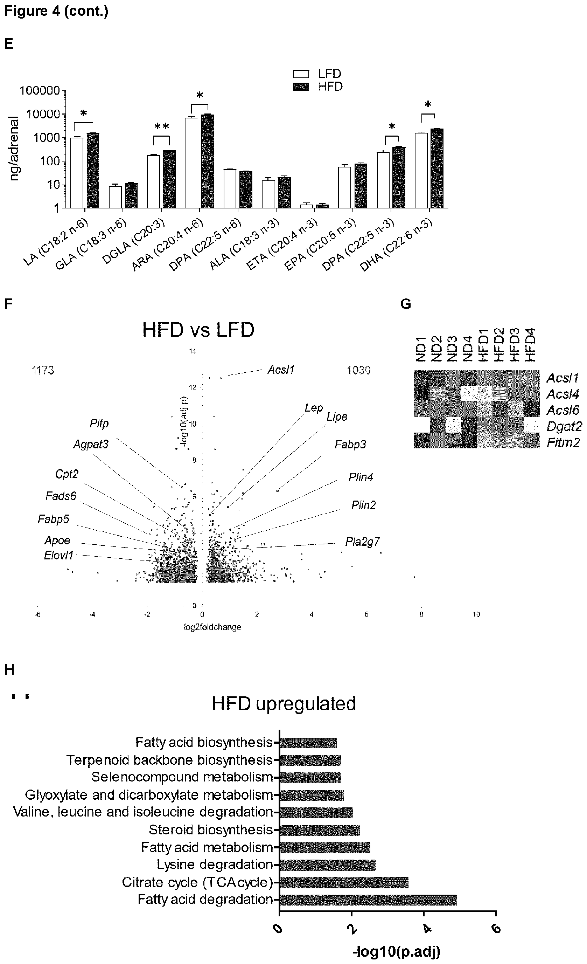

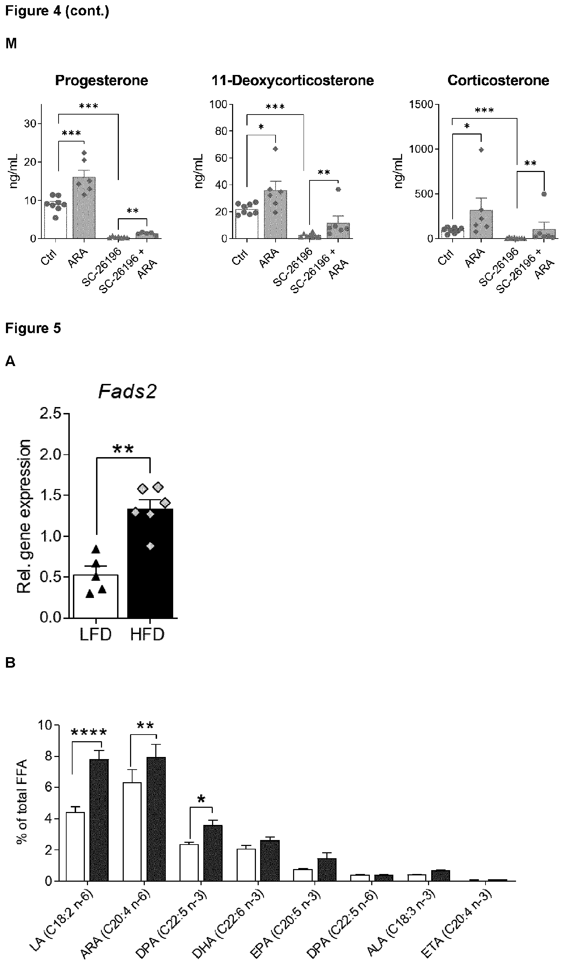

**Figure 4****:** A. Progesterone and corticosterone plasma levels were determined by LC-MS/MS in male C57BL/6 mice fed for 20 weeks a LFD or a HFD (n=8-12). B. Relative Fads2 expression in adrenal glands from lean and obese mice. Expression of *Fads2* in adrenal glands of LFD-fed mice was set to 1 (n=5-6). *Tbp* was used as a housekeeping gene. C. Representative immunofluorescence images of adrenal gland sections from LFD- and HFD-fed mice stained for FADS2 (red) and DAPI (blue) (one out of 5 mice per condition). Scale bar, 200 µm. A zoomed-in insert of each image is included at the right. D. Acyl chain profile of non-storage lipids analyzed by shotgun lipidomics. Lipids in adrenal glands from lean and obese mice were grouped according to the acyl chain within each lipid class (mol % is relative to the lipid class). Only the features with a mean abundance > 5 mol % are shown. The mean mol % ± standard deviation (SD) are shown (n=7-8). E. Acyl chain isomer in phospholipids in adrenal glands from LFD and HFD mice determined by High performance liquid chromatography (HPLC)-MS. Results are presented as ng/adrenal gland (n=5-6). F-H. RNA-Seq was performed in the adrenal cortex of mice fed for 20 weeks a LFD or HFD (n=4 mice per group). Volcano plot showing differentially expressed genes in HFD versus LFD mice and total number of significantly up- and downregulated genes. Genes related to lipid metabolism are depicted (F). Heatmap of differentially expressed genes involved in fatty acid processing (padj < 0.05) (G). EGSEA of RNA-Seq data in the adrenal cortex of HFD vs LFD mice showing 10 among most upregulated metabolic pathways (KEGG database) (H). M. Progesterone, 11-deoxycorticosterone and corticosterone levels in supernatants of primary adrenal cell cultures treated for 18 h with SC-26196 (10 µM) or DMSO (Ctrl) in the presence or absence of ARA (150 µM) (n=6-8). Data in A, B are shown as mean ± SD, and in E and M as mean ± SEM. * p-value<0.05;** p-value<0.01; *** p-value<0.001

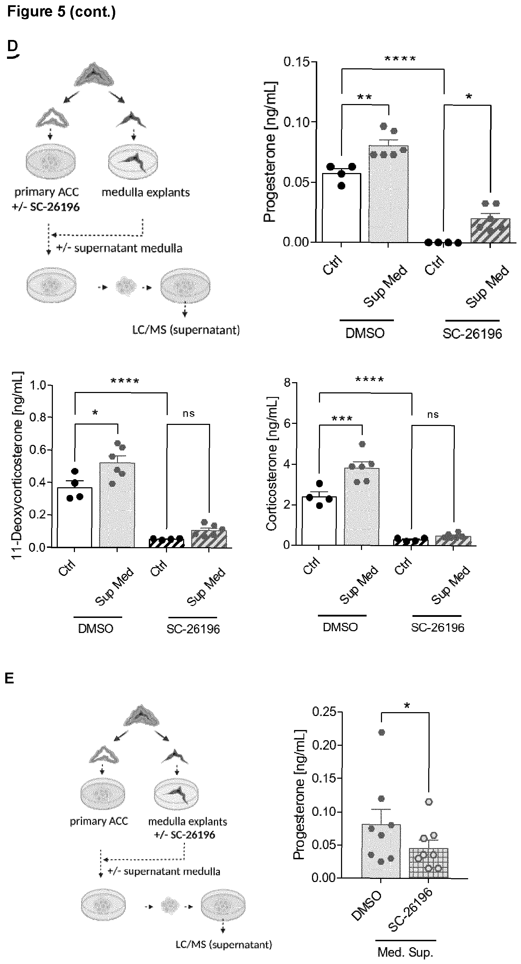

**Figure 5****:** A. Relative Fads2 expression in the adrenal medulla of mice fed for 20 weeks a LFD or HFD (n=5-6 mice per group). *Tbp* was used as a housekeeping gene B. FFA were measured in the adrenal medulla of mice fed for 20 weeks a LFD or HFD by HPLC-MS (n=3 mice per group). Results are presented as % of total FFA. D. Adrenocortical cells were treated for 6 h with SC-26196 (10 µM) or DMSO and then received medulla explant-conditioned or control medium for another 18 h. Steroids were measured in the adrenocortical cell supernatant by LC-MS/MS (n=4-6). E. Adrenal medulla explants were treated for 6 h with SC-26196 (10 µM) or DMSO, washed thoroughly and left another 18 h in culture. Their supernatant was then applied on adrenocortical cell cultures and 8 h later adrenocortical cell supernatants were collected and analyzed by LC-MS/MS (n=8). Data in A, B, D and E are shown as mean ± SEM * p-value<0.05; ** p-value<0.01; *** p-value<0.001; **** p-value<0.0001

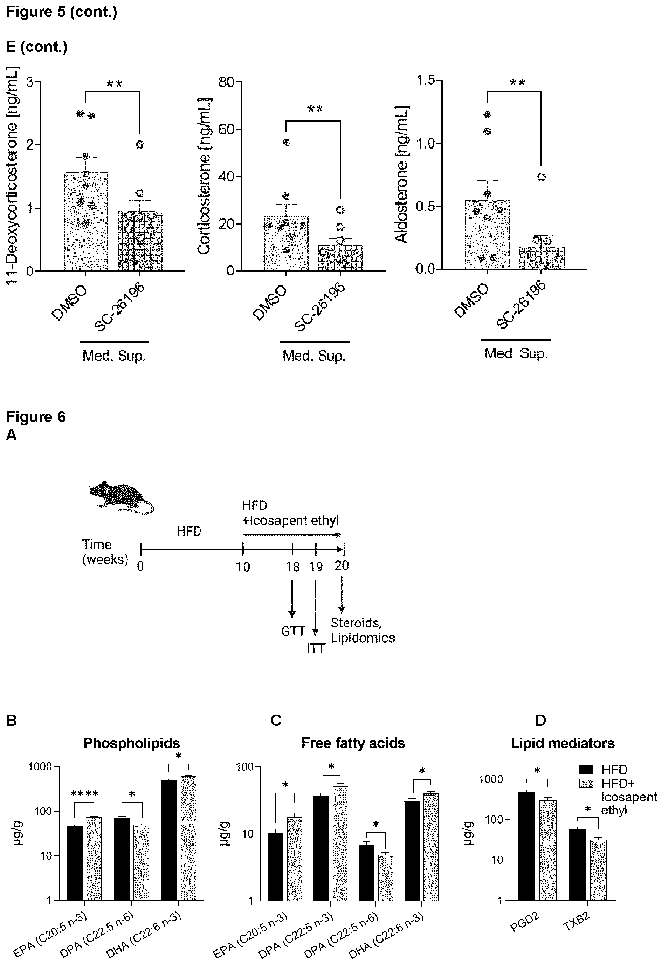

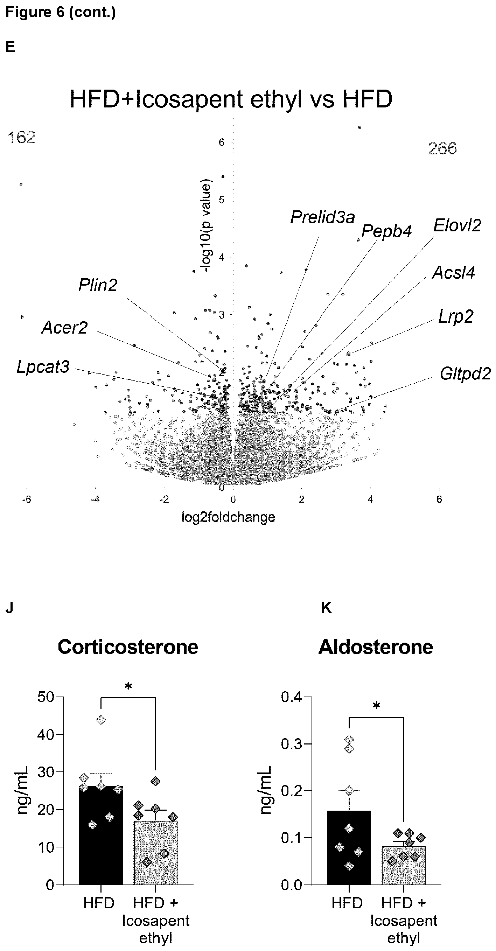

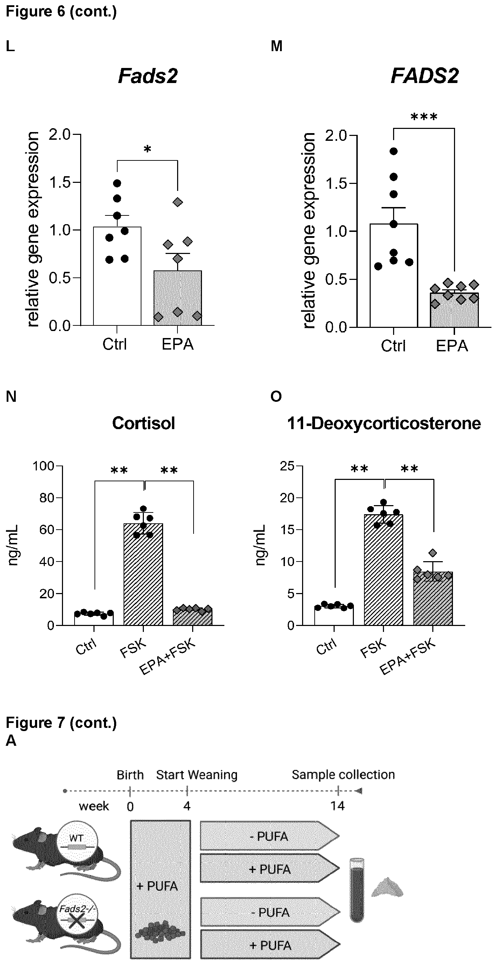

**Figure 6****:** A. Schematic representation of the experimental setup. B-D. Acyl chains of phospholipids (B), FFA (C) and lipid mediators (D) were measured by HPLC-MS in the adrenal glands of mice fed a HFD with or without icosapent ethyl (n=7 mice per group). E. Volcano plot based on RNA-Seq data in the adrenal cortex of mice, which received HFD supplemented with icosapent ethyl versus mice fed a HFD without icosapent ethyl (n=4 mice per group). J-K. Corticosterone and aldosterone plasma levels in mice, which received HFD without and with icosapent ethyl, measured by LC-MS/MS (n=7 mice per group). L-M. Primary CD31-CD45-adrenocortical cells (L) and NCI-H295R cells (M) were treated with 66 µM EPA for 18 or 48 h, respectively, and *Fads2* expression was determined by qPCR using *18S* expression as a housekeeping gene (n=7 in Land n=8 in M). N-O. NCI-H295R cells were treated for 48 h with 66 µM EPA and stimulated or not with forskolin (FSK,1 µM) in the last 24 h. Steroids were measured in the culture supernatant by LC-MS/MS (n=6). Data in B-D and L and M are presented as mean ± SEM, data in N and O as mean ± SD. * p-value<0.05; ** p-value<0.01; *** p-value<0.001; **** p-value<0.0001

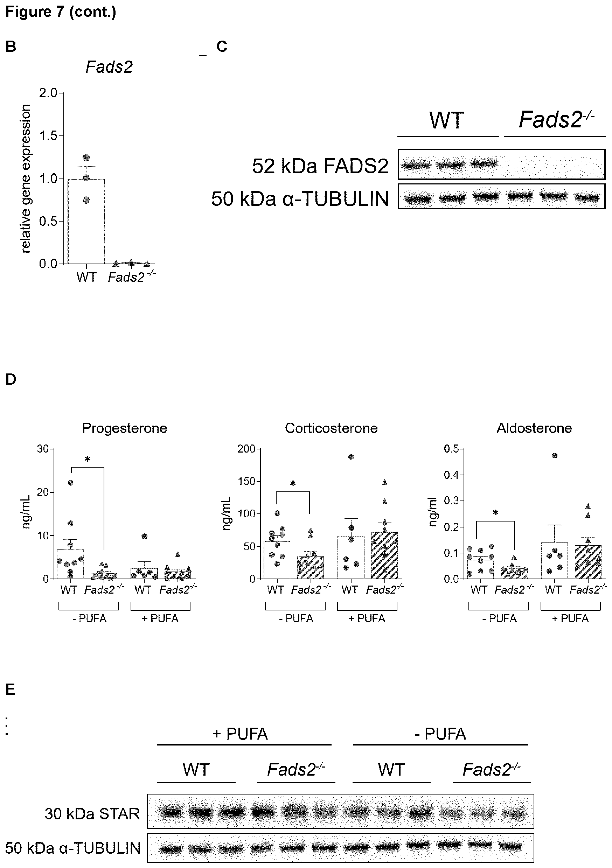

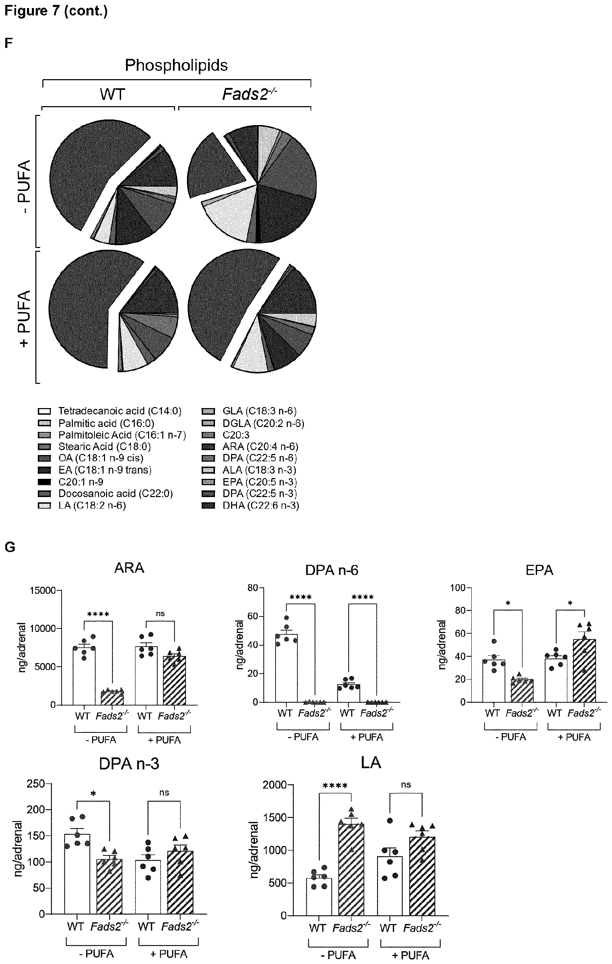

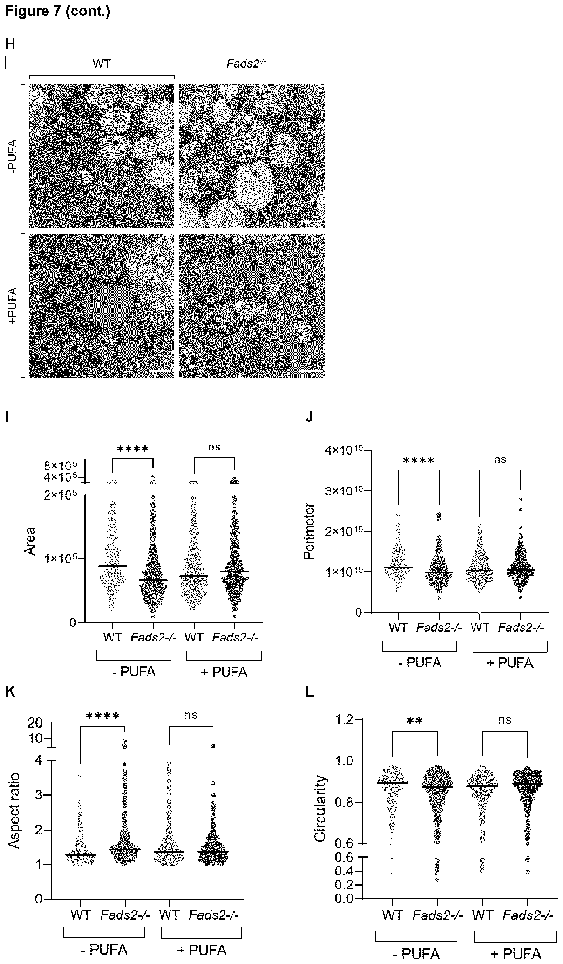

**Figure 7****:** A. Scheme demonstrating the experimental setup. B,C. FADS2 deletion efficiency in Fads2-/- mice. FADS2 expression was determined in adrenal glands of WT and Fads2-/- mice by qPCR using Tbp expression as a housekeeping gene (B) and western blot using α-Tubulin as a loading control (C) (n=3 mice per group). D. Progesterone, corticosterone and aldosterone levels in the plasma of WT and Fads2-/- mice fed a low-PUFA or PUFA-rich diet (n=6-9 mice per group). E. Western blot for StAR in adrenal glands of WT and Fads2-/- mice fed a low-PUFA or PUFA-rich diet. α-Tubulin was used as a loading control (n=3 mice per group). F,G Acyl chain composition of phospholipids in the adrenal gland of WT and Fads2-/- mice fed a low-PUFA or PUFA-rich diet (n=6 mice per group). Results are presented as % of all phospholipidic acyl chains (F) and as ng per adrenal gland (G). OA: Oleic acid, EA: Elaidic acid. H. Representative electron microscopy images of adrenocortical cells; adrenal glands of 2 WT and 2 Fads2-/- mice fed a low-PUFA and 1 WT and 1 Fads2-/- mice fed a PUFA-rich diet were imaged (magnification 6,800x). Scale bar, 1 µM. Asterisks (*) depict lipid droplets, arrowheads (>) depict mitochondria. I-L. Quantification of mitochondrial area (I), perimeter (J), aspect ratio (K) and circularity (L) in WT and Fads2-/- mice fed a low-PUFA or PUFA-rich diet (in total 198-429 mitochondria were quantified in 2 WT and 2 Fads2-/- mice fed a low-PUFA and 1 WT and 1 Fads2-/- mice fed a PUFA-rich diet). Data in B, D and G are shown as mean ± SEM. * p-value<0.05; ** p-value<0.01; **** p-value<0.0001

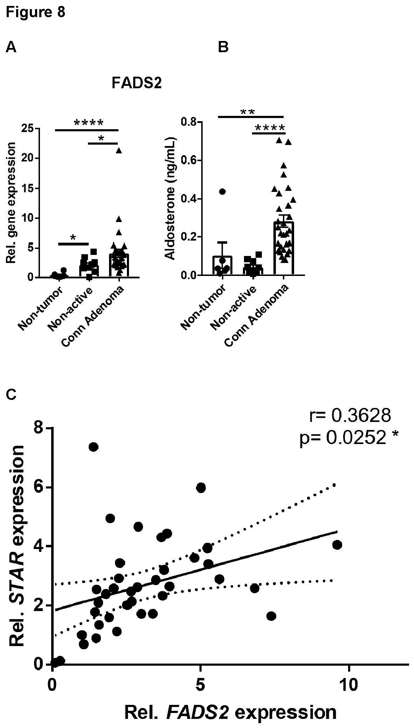

**Figure 8****:** A. FADS2 expression was determined in non-tumorous adrenocortical tissue from pheochromocytoma patients (n=6 patients), non-active adenomas (n=10 patients) and aldosterone-producing adenomas (Conn adenomas) (n=30 patients) by qPCR using 18S as a housekeeping gene. B. Pre-operative aldosterone plasma levels in the same patients as in (A) were measured by LC-MS/MS. C. Correlation of FADS2 with STAR expression in aldosterone- and cortisol-producing adrenocortical adenomas from 30 and 7 patients, respectively. FADS2 and STAR expression was determined by qPCR using 18S as a housekeeping gene. Data in A and B are presented as mean ± SEM. * p-value<0.05; ** p-value<0.01; **** p-value<0.0001

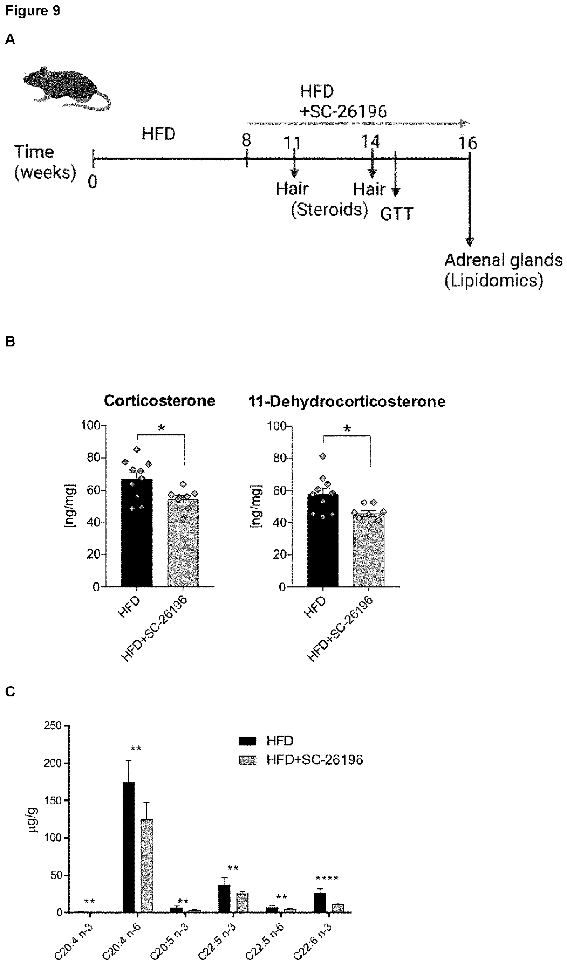

**Figure 9****:** A. Schematic representation of the experimental setup. B-C. Corticosterone and 11-dehydrocorticosterone levels were determined by LC-MS/MS at feeding week 14 in hair of HFD mice receiving or not for 6 weeks SC-26196. Steroid concentrations were normalized to hair weight (n=8-10 mice per group). C. FFA were measured by HPLC-MS in the adrenal glands of mice fed a HFD with or without SC-26196 (n=8-10 mice per group). Data are presented as mean ± SEM, * p-value<0.05; ** p-value<0.01; **** p-value<0.0001

### EXAMPLES

### Example 1: Icosapent-ethyl and eicosapentaenoic acid inhibit glucocorticoid production:

Mice were fed with a high-fat diet for 10 weeks followed by 10 weeks of the high-fat diet with or without icosapent-ethyl (1 .125 g/kg diet weight, 180 mg/kg body weight). It is shown that Icosapent-ethyl lowers glucocorticoid levels (Figure 1A). Furthermore, in vitro studies showed that eicosapentaenoic acid strongly reduced glucocorticoid production in human and primary murine adrenocortical cells (Figure 1B, C). This effect of eicosapentaenoic acid can be attributed to the inhibition of fatty acid desaturase 2 (FADS2). FADS2 mediates the synthesis of arachidonic acid, thereby promoting glucocorticoid production. Based on these findings, the invention shows that icosapent-ethyl can be used to lower cortisol levels in obese patients (Figure 2).

### Example 2: Upon treatment with icosapent-ethyl the production and function of cortisol are reduced in patients with obesity:

Cortisol is measured in patients before and after treatment with icosapent-ethyl. It is expected that upon treatment with icosapent-ethyl the production and function of cortisol are reduced in patients with obesity. The experiment is ongoing.

### Example 3: FADS2 determines the mitochondrial lipidome and is required for steroidogenesis in adrenocortical cells:

As key steps of steroidogenesis take place in mitochondria (14), it was first interrogated to which extent the lipid composition of mitochondria affects steroidogenesis in adrenocortical cells. Δ-6-desaturase FADS2 was chosen as a target to modulate mitochondrial lipid composition in adrenocortical cells, since this gene is highly expressed in the adrenal gland compared to other tissues and organs (Figure 3A) and its expression is higher in steroidogenic cells (CD31-CD45-) compared to endothelial cells (CD31+) and leukocytes (CD45+) of the adrenal cortex (Figure 3B). In fact, the adrenal gland and the liver were the organs with the highest FADS2 mRNA and protein expression among many different tested organs and tissues (Figure 3A). Moreover, FADS2 is the rate-limiting enzyme in the synthesis of PUFAs, which are important components of functional and storage lipids (15, 16). FADS2 catalyzes the conversion of the omega-6 linoleic acid (18:2 n-6, LA) to γ-linolenic acid (18:3 n-6, GLA), and the omega-3 α-linolenic acid (18:3 n-3, ALA) to stearidonic acid (18:4 n-3)(17-19).

NCI-H295R adrenocortical carcinoma cells were treated for 18 h with the FADS2 inhibitor SC-26196 and analyzed the lipidome of mitochondrial fractions (identified by Succinate dehydrogenase B (SDHB) expression) by liquid chromatography tandem mass spectrometry (LC-MS/MS). FADS2 inhibition reprogramed the mitochondrial lipidome, as shown by principal component analysis (PCA) (Figure 3C) and strongly altered the mitochondrial lipid composition at individual species level (Figure 3D). The abundance of phosphatidylcholines (PC) and phosphatidylethanolamines (PE) containing PUFA acyl chains, such as PC 16:0_20:4, PC 20:1_20:3, PC 20:1_20:4, PE 16:0_20:4, PE 18:1_20:5 and PE 16:1_20:3 was reduced in SC-26196-treated cells while conversely lipids with saturated or monounsaturated acyl chains, such as PE 14:0_16:1, PE 16:1_16:1, phosphatidylinositol (PI) PI 16:0_16:0, PI 16:0_16:1, PI 16:0_18:1, PI 18:1_16:1, PI 18:0_18:1, PI 18:0_20:1, PI 18:0_22:1 and phosphatidylglycerol (PG) PG 16:1_16:1 were increased (Figure 1D). Cardiolipins, a unique component of the inner mitochondrial membrane (IMM) (20), remained unaffected (not shown).

PC and PE are major lipid components of mitochondrial membranes and therefore changes in their consistency are likely to influence mitochondrial physiology (21, 22). Indeed, FADS2 inhibition decreased the mitochondrial load, mitochondrial membrane potential and oxygen consumption rate (OCR) in primary adrenocortical cells (Figure 3E-G). Electron transport chain (ETC)-dependent mitochondrial bioenergetics are intimately linked with and required for steroidogenesis (14, 23-26). Hence, the observed reduction of the mitochondrial membrane potential (Figure 3F) and OCR (Figure 3G) is expected to lead to reduced steroidogenesis. Along this line, we asked whether cholesterol transport into mitochondria, which is the rate-limiting step of steroidogenesis, is affected by FADS2 inhibition. Cholesterol import into mitochondria involves the interaction between StAR, Voltage-dependent anion channel (VDAC) and Translocator Protein, 18 kDa (TSPO) (14). StAR shuttles from the cytoplasm to the outer mitochondrial membrane (OMM), transferring cholesterol to a complex, which is assembled by VDAC and TSPO at the OMM - IMM contact sites, called transduceosome (14, 26). In adrenocortical steroidogenic cells, cholesterol taken up from circulating lipoproteins or synthesized in the endoplasmic reticulum (ER), is mainly stored in the form of CE in lipid droplets, from where it is transferred by StAR and the transduceosome into the mitochondria for pregnenolone synthesis, the first steroid to be synthesized in the steroidogenic cascade (26). In order to examine whether FADS2 inhibition affects cholesterol uptake in the mitochondria we analyzed the cholesterol content of mitochondrial (SDHB positive) fractions of NCI-H295R cells treated or not with SC-26196 upon stimulation with forskolin. Forskolin treatment triggered a strong increase in mitochondrial cholesterol levels in control- but not SC-26196-treated cells (Figure 3H). In accordance, cholesterol and CE accumulated in the lipid droplet (Hormone sensitive lipase (HSL) positive) fractions in SC-26196-treated cells.

Reduced mitochondrial bioenergetics (Figures 3F,G) and cholesterol mitochondrial import (Figure 1H) in SC-26196-treated cells are expected to lead to impaired steroidogenesis. To verify this, we analyzed steroid hormone production by LC-MS/MS in primary adrenal cell cultures treated with SC-26196 and subsequently stimulated with ACTH. ACTH efficiently increased progesterone, 11-deoxycorticosterone, corticosterone and aldosterone production, while FADS2 inhibition abolished their production (Figure 3I), without affecting cell viability (data not shown). FADS2 inhibition also reduced progesterone and 11-deoxycortisol production in forskolin-treated NCI-H295R cells. The effect of FADS2 inhibition on steroidogenesis was not due to decreased mRNA expression of StAR or steroidogenic enzymes, Cytochrome P450 Family 11 Subfamily A Member 1 (Cyp11a1, Cholesterol side-chain cleavage enzyme), 3β-hydroxysteroid dehydrogenase type 2 (3β-Hsd2), Cyp11b1 or Cyp11b2; in fact, their expression was increased upon FADS2 inhibition, perhaps as a compensatory response to low steroid hormone synthesis. These data indicate that increased expression of steroidogenic enzymes is not sufficient for successful steroidogenesis when FADS2 is blocked, thereby underscoring the importance of FADS2-dependent PUFA synthesis for steroidogenesis. In accordance, FADS2 overexpression increased 11-deoxycorticosterone and aldosterone production in forskolin-treated NCI-H295R cells substantiating the promoting effect of FADS2 on steroidogenesis.

### Example 4: High-fat diet reprograms the adrenal lipidome and increases glucocorticoid production:

To investigate whether FADS2 could drive increased adrenocortical hormone production in vivo. As an experimental model high-fat diet (HFD)-induced obesity in mice were used, which was previously shown to lead to increased corticosterone and aldosterone production and adrenocortical hyperplasia (7). Wild-type (WT) C57BL6/J mice were fed for 20 weeks a low-fat diet (LFD, 10 % kcal deriving from fat) or a HFD (60 % kcal deriving from fat). HFD feeding substantially increased the body weight of the animals (data not shown), as previously reported in a large number of studies (27-30). As expected, progesterone and corticosterone plasma levels were elevated in HFD compared to LFD mice (Figure 4A). ACTH plasma levels were also increased in obese mice (not shown), standing in accordance with the notion that chronic inflammation and dysmetabolism may lead to chronic activation of the hypothalamic-pituitary-adrenal (HPA) axis (7, 31, 32). Adrenal gland weights did not change with obesity. Here, the lipidomic changes in the adrenal glands of obese mice were investigated and whether these changes may contribute to the increased steroidogenesis in an autonomous fashion.

FADS2 expression was elevated at mRNA and protein level in the adrenal glands of HFD-fed mice (Figure 4B,C), while its expression did not change in other organs, such as the liver or the brain. To understand its role in regulating the adrenocortical lipidome, the lipidome of the adrenal gland was investigated by shotgun lipidomics which revealed reprograming of the whole lipidome, i.e. both non-storage lipids and storage lipids upon HFD feeding. Non-storage, i.e. membrane lipids include phospholipids and their ether-linked versions (PC, PE, PC O-, PE O-, PI, PG, phosphatidylserine (PS), phosphatidate (PA)), lysolipids (lyso-phosphatidate (LPA), lysophosphatidylcholine (LPC), phosphatidylethanolamine (LPE), LPE O-, lyso-phosphatidylglycerol (LPG), lyso-phosphatidylinositol (LPI), lyso-phosphatidylserine (LPS)), sphingomyelin (SM), diacylglycerol (DAG), cardiolipins (CL), ceramides and hexosylceramides (HexCer). Storage lipids comprise CE and triacylglycerol (TAG) (33). Whole and non-storage lipidomes of adrenal glands from lean and obese mice segregated along the first principal component (PC1) and the topology of the samples was strikingly similar, while storage lipids regrouped with statistical significance along the second principal (PC2), suggesting that non-storage lipids were mainly responsible for the grouping of the whole lipidome according to the diet.

Yet, storage lipids (TAG and CE) constituted the largest portion of the identified lipids in adrenal glands (86.98 ± 5.43 % in LFD mice and 90.12 ± 1.93 % in HFD mice), while non-storage lipids accounted for 13.02 ± 5.22 % and 9.87 ± 1.89 % of all identified lipids in the adrenal glands of mice under LFD and HFD, respectively. The total amounts of storage and non-storage lipids did not substantially differ in the adrenal glands of lean and obese mice. Likewise, lipid classes did not significantly differ between the adrenal glands of lean and obese mice. CE as well as non-esterified cholesterol were also not significantly increased in the adrenal glands of obese mice.

Our data show that it is not the lipid amount but the total acyl chain length and degree of unsaturation of storage and non-storage lipids that significantly changed with obesity. TAG species with 50 to 54 carbon atoms and 2 to 4 acyl chain double bonds were highly abundant (mol % > 10). Adrenal glands of HFD mice exhibited a significant increase in long (with a total acyl chain length of 54 carbon atoms) and unsaturated (with 4, 5 and more than 6 double bonds) TAG species. In contrast, adrenal glands from LFD-fed mice showed a larger amount of TAG species containing shorter acyl chains (with 48 and 50 carbon atoms) with 1 or 2 double bonds. To determine whether the diet affected the overall mean weighted length and unsaturation level of lipids, we calculated the double bond index (DBI) and the length index (LI). Storage lipids in the adrenal glands of obese mice showed a significantly higher DBI compared to lean mice. Hence, upon HFD feeding TAG in the adrenal glands became overall more unsaturated.

Most non-storage lipids contained a cumulative of 34 to 38 carbon atoms and 4 double bonds in their acyl chains. Similarly to what was found for TAG, adrenal glands from HFD mice showed a significant increase in longer and more unsaturated (with 4 double bonds) non-storage lipids, while shorter and mono-unsaturated non-storage lipid species were more abundant in the adrenal glands of LFD-fed mice. In accordance, membrane lipids in the adrenal glands of HFD-fed mice had significantly higher DBI and LI compared to LFD-fed mice, indicating that non-storage (membrane) lipids were longer and more unsaturated in the adrenal glands of obese mice.

Strikingly, 20:4 was one of the most abundant acyl chain in PC, PC O-, PE, PE O-, PG and PI and the amount of PC, PE and PG lipids containing 20:4 was higher in the adrenal glands of obese compared to lean mice (Figure 4D). In contrast, long chain PUFAs other than 20:4, such as 22:4, 22:5 and 22:6, were less abundant (Figure 4D). Accordingly, the phospholipids PC 18:0_20:4 and PE 18:0_20:4 were highly abundant and higher in the adrenal glands of HFD mice .

Isomer analysis showed that arachidonic acid (20:4 n-6, all-cis-5,8,11,14-eicosatetraenoic acid, ARA) but not eicosatetraenoic acid (20:4 n-3, all-cis-8,11,14,17-eicosatetraenoic acid, ETA) was the abundant 20:4 isomer form (Figure 4E), in agreement with previous reports showing very little ETA in the tissue lipidome (34). Furthermore, docosapentaenoic acid (22:5 n-3, DPA) and docosahexaenoic acid (22:6 n-3, DHA), were more abundant in adrenal gland phospholipids of obese mice, while eicosapentaenoic acid (20:5 n-3, EPA) was not (Figure 4E). Given that FADS2 is the rate-limiting enzyme for ARA, DPA and DHA synthesis, the increased amounts of these phospholipidic acyl chains in the adrenal glands of HFD mice could be the result of an enhanced adrenal FADS2 expression in obese mice (Figure 4B,C).

To correlate the observed shift at the lipidome level with transcriptional changes, we performed RNA-Seq in the adrenal cortex of LFD and HFD mice that revealed 2,203 differentially expressed genes, out of which 1,030 were up- and 1,173 were downregulated by HFD compared to LFD feeding (Figure 4F). Several genes involved in lipid metabolism were highly upregulated, such as Acyl-CoA synthetase long chain family member 1 (Acsl1), Fatty acid binding protein 3 (Fabp3), Lipase e (Lipe) and Perilipin 4 (Plin4), or downregulated, such as Phospholipid transfer protein (Pltp), Fads6, 1-Acylglycerol-3-phosphate O-acyltransferase 3 (Agpat3) or Carnitine palmitoyltransferase (Cpt2) in the adrenal cortex of HFD mice (Figure 4F). Fads2 was also upregulated in the adrenal cortex of HFD- compared to LFD-fed mice (log2FC=1.244, adjp value=0.0117), as were several other genes involved in fatty acid processing, such as Acsl4, the most highly expressed Acsl isoform in the adrenal gland, Diacylglycerol O-acyltransferase 2 (Dgat2) and Fat storage-inducing transmembrane protein 2 (Fitm2) (Figure 4G). Ensemble of Gene Set Enrichment Analysis (EGSEA) showed that fatty acid metabolism, steroid biosynthesis and TCA cycle pathways were among the most upregulated pathways in the adrenal cortex of HFD mice (Figure 4H).

Summarizing, these findings demonstrate that HFD-induced obesity is associated with lipidomic transformation of the adrenal gland, and particularly enhanced PUFA synthesis, increased

FADS2 expression and transcriptional changes promoting increased PUFA metabolism and mitochondrial bioenergetics in the adrenal cortex. A particularly abundant PUFA in adrenal PC and PE is ARA (Figure 4D). ARA was previously shown to promote steroidogenesis and potentially be required for cholesterol import into mitochondria (35, 36). We found that FADS2 is required for the incorporation of ARA acyl chains in mitochondrial PC and PE (Figure 3D). Hence, we tested whether ARA supplementation can counteract the inhibitory effect of FADS2 inhibition on steroidogenesis. Indeed, ARA partially restored progesterone, 11-deoxycorticosterone and corticosterone production in primary adrenocortical cells treated with SC-26196 (Figure 4M). The fact that ARA only partially restored steroidogenesis could be due to insufficient lipidomic remodeling achieved by ARA supplementation under the used experimental conditions or may indicate the requirement of additional PUFAs for full restorage of steroidogenesis under FADS2 inhibition. These data imply that intra-adrenal FADS2-dependent ARA synthesis is required for adrenocortical steroidogenesis.

### Example 5: Medulla - derived PUFA promote adrenocortical steroidogenesis:

The adrenal gland consists of the medulla and the cortex. Early reports showed that chromaffin (medullary) cells may release ARA (37). It was investigated whether FADS2-mediated PUFA synthesis in the medulla could influence adrenocortical steroidogenesis. Fads2 expression was higher in the adrenal medulla of obese compared to lean mice (Figure 4C, 5A). In addition, free LA (C18:2 n-6) and ARA were increased in the medulla of obese compared to lean animals (Figure 5B). Additionally, free ARA was among the most abundant free fatty acid (FFA) in the supernatant of medulla organ cultures (Figure 5C). Together with our finding that ARA supplementation increased steroidogenesis in adrenocortical cells (Figure 4M), these data suggest that ARA may mediate a medulla-cortex interaction promoting adrenocortical steroidogenesis.

In order to investigate how PUFAs from chromaffin cells may affect steroidogenesis in adrenocortical cells, we applied supernatants from medulla explants onto primary adrenocortical cell cultures. Medulla-conditioned supernatants increased progesterone, 11-dehydrocorticosterone and corticosterone production in primary adrenocortical cultures compared to control medium (Figure 5D). This effect was attenuated when medulla explants were treated with the FADS2 inhibitor SC-26196, underscoring the importance of medullar PUFA synthesis in the medulla-cortex interaction (Figure 5E). However, treatment of adrenocortical cells with SC-26196 diminished steroid synthesis in cells receiving medulla-conditioned medium (Figure 5D), suggesting that despite the fact that adrenocortical cells may take up medulla-deriving PUFA, it is mainly endogenous synthesis of PUFA in adrenocortical cells, which is required for steroidogenesis.

### Example 6: Dietary supplementation with icosapent ethyl reduces corticoid levels in obese mice:

To further elaborate on the requirement of FADS2-dependent PUFA synthesis for adrenocortical steroidogenesis, it has been investigated whether enhanced activation of the FADS2-ARA axis can be regulated by dietary interventions. In general, increased intake or elevated blood levels of omega-3 lipids are associated with health benefits, while an omega-6/omega-3 imbalance is a risk factor for chronic disease (38, 39). Icosapent ethyl, a highly purified and stable EPA ethyl ester, is approved as an adjunct to diet to reduce triglyceride, non-high-density lipoprotein (HDL) and apolipoprotein B levels and to lower the risk of cardiovascular events in hypertriglyceridimic patients, as shown by the recent Reduction of Cardiovascular Events with Icosapent Ethyl-Intervention Trial (REDUCE-IT) and other clinical studies (40-42). It has been investigated whether dietary supplementation with icosapent ethyl can lower corticosterone and aldosterone levels in mice with diet-induced obesity. To this end, WT C57BL/6J mice were fed for 10 weeks a HFD and another 10 weeks with HFD containing or not 1.125 g/kg icosapent ethyl (corresponding to approx. 180 mg/kg of animal weight) (Figure 6A). Lipidomic analysis in the adrenal glands of these mice showed increased EPA incorporation in the phospholipids of icosapent ethyl-treated mice, as well as higher DHA and lower omega-6 DPA levels in adrenal phospholipids (Figure 6B). Accordingly, free EPA, omega-3 DPA and DHA were increased and free omega-6 DPA was decreased in the adrenal glands of mice receiving icosapent ethyl (Figure 6C). Moreover, analysis of lipid mediators (oxylipins) showed reduced prostaglandin D2 (PGD2) and thromboxane B2 (TXB2) levels in the adrenal glands of icosapent ethyl-treated mice (Figure 6D). Hence, dietary supplementation with icosapent ethyl in obesity transforms the adrenal lipidome towards increased omega-3 lipid content and reduced levels of pro-inflammatory lipid mediators.

The lipidomic alterations caused by icosapent ethyl dietary supplementation were accompanied by transcriptional changes in the adrenal cortex, as shown by RNA-Seq, with 266 genes being up- and 162 genes being down-regulated (Figure 6E). Among the differentially expressed genes several lipid metabolism-associated genes, such as Acsl4 and Elovl2 were up-regulated, while others, such as Plin2 and lysophosphatidylcholine acyltransferase 3 (Lpcat3) were downregulated (Figure 6E). GSEA analysis showed negative enrichment of genes related to fatty acid metabolism, cholesterol homeostasis and steroid biosynthesis in the adrenal cortex of icosapent ethyl-treated mice, pathways which were upregulated in the adrenal cortex of HFD compared to LFD mice. In addition, genes related to mitochondrial envelope formation, respiratory electron transport, oxidative phosphorylation and TCA cycle were negatively enriched in the adrenal cortex of icosapent ethyl-receiving mice, again standing in stark contrast to the upregulation of these pathways in the adrenal glands of HFD versus LFD mice. Importantly, icosapent ethyl dietary supplementation reduced corticosterone and aldosterone plasma levels in obese mice (Figure 6J,K) without altering ACTH plasma levels. These data collectively demonstrate that dietary administration of icosapent ethyl in obesity reduces adrenocortical steroidogenesis in an HPA axis-independent fashion. Instead, it is associated with lipidomic and transcriptional reprograming of the adrenal cortex, suggesting an intra-adrenal regulation of corticosteroid production. In support of these findings, EPA treatment of primary adrenocortical and NCI-H295R cells down-regulated FADS2 expression (Figure 6L,M) and reduced cortisol and 11-deoxycortisol production in forskolin-stimulated NCI-H295R cells, and ACTH-stimulated primary adrenocortical cells (Figure 6N and O), further substantiating that FADS2 can determine steroidogenesis.

### Example 7: FADS2 deficiency perturbs adrenal gland function:

To further confirm the role of the FADS2-dependent PUFA synthesis in adrenocortical function, we used Fads2 knockout (Fads-/-) mice, which were kept for 10 weeks under a diet with low or high PUFA concentration (Figure 7A). FADS2 deficiency was verified in the adrenal gland at mRNA and protein level (Figure 7B,C). Adrenal glands from Fads2-/- mice did not show any obvious abnormalities in size or macroscopic structure compared to littermate WT mice fed a PUFA-rich or low-PUFA diet. However, progesterone, corticosterone and aldosterone plasma concentrations were reduced in Fads2-/- mice fed the low-PUFA diet, but not in mice fed the PUFA-rich diet (Figure 7D). Reduced corticosteroid production was not due to reduced ACTH levels, i.e. WT and Fads2-/- mice fed with either diet displayed similar ACTH plasma levels. In accordance, StAR expression was reduced in Fads2-/- mice, which were kept under low-PUFA diet but not in mice fed the PUFA-rich diet (Figure 7E). Expression of other steroidogenic genes (Cyp11a1, 3β-Hsd2, Cyp11b1, Cyp11b2) was not affected by FADS2 deficiency.

As outlined above, FADS2-mediated ARA synthesis is crucial for adrenal steroidogenesis. Lipidomic analysis showed that ARA abundance in adrenal phospholipids of WT mice was not affected by feeding with the low-PUFA diet (Figure 7F, G), suggesting that the ARA content in adrenal phospholipids is not affected by dietary ARA uptake when FADS2 is expressed. However, in Fads2-/- mice fed the low-PUFA diet the ARA content in adrenal phospholipids was strongly reduced compared to WT mice fed the same diet, but not in mice fed a PUFA-rich diet (Figure 7F,G). DPA (C22:5 n-6) levels in phospholipids were also diminished in the adrenal glands of Fads2-/- mice independently of the diet (Figure 7F). The levels of EPA and DPA (C22:5 n-3) were reduced in the adrenal glands of FADS2-deficient mice receiving a low-PUFA diet (Figure 7F,G), although to a lesser extent than the omega-6 fatty acids ARA and DPA (C22:5 n-6), suggesting that FADS2 may rather promote the synthesis of omega-6 fatty acids. In accordance, the PUFA precursor LA increased in the adrenal glands of Fads2-/- mice fed the low-PUFA diet presumably due to its diminished use for PUFA synthesis (Figure 7F,G).

Moreover, adrenocortical cells of Fads2-/- mice fed the low-PUFA diet had larger lipid droplets compared to WT mice receiving the same diet, as shown by electron microscopy (Figure 7H). This stands in accordance with the observed reduced steroid hormone production (Figure 7D), hence, the attenuated use of CE for steroidogenesis, which are thus accumulating in cells forming larger lipid droplets. Adrenocortical cells of Fads2-/- mice fed the low-PUFA diet also displayed fewer, smaller and less circular mitochondria (Figure 7H-L), indicating perturbed mitochondrial function, thus standing in agreement with impaired steroidogenesis. Altogether, these data further indicate that FADS2-dependent PUFA synthesis determines the lipidomic landscape, mitochondrial structure, lipid droplet load and steroidogenesis in adrenocortical cells.

### Example 8: FADS2 expression correlates with the expression of steroidogenic genes in the human adrenal gland and is increased in aldosterone-producing adenomas:

In order to validate the significance of our findings in the human adrenal gland, we mined the public GEPIA2 database (http://gepia2.cancer-pku.cn/#index) (43) for steroidogenic genes and analyzed their expression in correlation with the expression of FADS2. FADS2 expression positively correlated with the expression of STAR, Voltage-dependent anion channel 1 (VDAC1), CYP11A1, Steroidogenic Factor-1 (SF-1) and protein kinase A (PKA) catalytic subunit A (PRKACA) in the human adrenal gland. VDAC1 interacts with StAR at the outer mitochondrial membrane, facilitating cholesterol transport into mitochondria (44). CYP11A1 catalyzes the side-chain cleavage reaction, which is the first step of steroidogenesis generating pregnenolone (45). PRKACA mediates PKA activation, which triggers steroidogenesis, and is involved in adrenocortical pathologies with ACTH-independent hypercortisolism, such as adrenocortical adenomas, carcinomas or bilateral adrenocortical hyperplasias (BAH) (46). SF-1 is the major regulator of steroidogenesis-related genes (47). The correlation of the expression of FADS2 with the expression of genes that play a critical role in the initiation of steroidogenesis in the human adrenal gland underscores the relevance of our findings. Based on GEPIA2 data, FADS2 also positively correlates with SF-1 and PRKACA expression in adrenocortical carcinoma.

Along this line, we investigated the changes in FADS2 expression in human adrenal tumors. FADS2 expression was analyzed in non-tumorous adrenocortical tissue, non-functioning adrenocortical adenomas (hormonally inactive) and aldosterone-producing adenomas (Conn adenomas) and was found to be significantly higher in aldosterone-producing adenomas compared to both, non-tumorous adrenocortical tissue and non-functioning adenomas (Figure 8A). Of note, *FADS2* expression was also elevated in hormonally inactive tumors compared to non-tumorous adrenocortical tissue (Figure 8A), suggesting a potential role of FADS2 in adrenocortical tumorigenesis. Increased FADS2 expression in Conn adenomas was associated with enhanced pre-operative aldosterone levels in these patients compared to patients of the two other groups (Figure 8B). Additionally, FADS2 expression positively correlated with STAR expression in aldosterone- and cortisol-producing adrenocortical adenomas (Figure 8C). These data collectively support that FADS2 is not only required for steroidogenesis in the human adrenal gland but may also promote increased steroid production in adrenocortical adenomas.

### Example 9: FADS2 inhibition reduces corticosteroid levels in obese mice

In order to validate the role of FADS2 in adrenocortical steroidogenesis in obesity, we treated HFD mice with the FADS2 inhibitor SC-26196 and assessed corticosteroid production. WT C57BL/6J mice were fed for 8 weeks a HFD and another 8 weeks with HFD containing or not 0.625 g/kg SC-26196 (corresponding to approx. 100 mg/kg of animal weight) (Figure 9A). We decided to determine corticosteroid levels at 2 different time points, i.e. 3 and 6 weeks after SC-26196 treatment start, during the course of the HFD feeding in hair, instead of plasma, since hair cutting causes minimal stress to mice compared to blood retrieval (Figure 9A). Notably, corticosteroid levels in newly grown hair (2-3 mm above the skin) reflect the cumulative corticosteroid production over a period of 3-4 weeks (93, 94). Already at 3 weeks after SC-26196 treatment start, levels of 11-dehydrocorticosterone in newly-grown hair were reduced in HFD mice receiving the inhibitor compared to control mice. 11-dehydrocorticosterone is the inactive form of corticosterone, which is converted to the latter by 11β-HSD1 in tissues. Hence, reduced availability of 11-dehydrocorticosterone can lead to lower (active) corticosterone levels in tissues. At 6 weeks after SC-26196 treatment start, both 11-dehydrocorticosterone and corticosterone levels were reduced in the hair of mice receiving the FADS2 inhibitor compared to control mice (Figure 9B). Lower corticosteroid production associated with reduced free PUFA, including ARA, levels in the adrenal glands of SC-26196 treated mice (Figure 9C). These findings demonstrate that FADS2 inhibition in established obesity can alter the PUFA content of the adrenal glands and downregulate adrenocortical steroidogenesis.

### Discussion of the Examples:

Chronically elevated cortisol levels can have deleterious consequences, including disturbed lipid and glucose metabolism, immunodeficiency and neurological disorders (5, 31). Increased aldosterone levels promote hypertension and increase the risk of cardiovascular disease (4). Moderately elevated corticoid levels in obese subjects contribute to complications such as insulin resistance and hypertension, and can thus substantially affect life quality but are rarely treated (6, 9, 10). The mechanisms underlying derailed adrenocortical hormone production in obesity are incompletely understood. Herein, we investigated to which extent the lipidomic landscape of the adrenal gland affects its steroidogenic function. We analyzed the adrenal lipidome of lean and obese mice and found that a high content of phospholipids in longer and more unsaturated lipids is associated with increased steroidogenesis in obese mice. ARA is a particularly abundant acyl chain in adrenal phospholipids and is increased with obesity. We identified FADS2, the rate-limiting enzyme for PUFA synthesis, as a key determinant for the adrenal lipidome and steroidogenic function. FADS2 is highly expressed in the adrenal gland compared to other tissues and increases in the adrenal gland of obese animals. Moreover, we propose that not only adrenocortical FADS2-mediated ARA synthesis, but also medulla-derived ARA may affect adrenocortical steroidogenesis.

The importance of the FADS2-ARA axis for steroidogenesis was validated in Fads2-/- mice, which displayed lower corticosterone and aldosterone plasma levels compared to WT littermates when receiving for 10 weeks a low-PUFA diet, while corticoid levels were restored by a PUFA-rich diet. In accordance, adrenocortical cells in Fads2-/- mice fed a low-PUFA diet had larger lipid droplets and disturbed mitochondria. Along the same line, in vitro treatment of adrenocortical cells with SC-26196, a FADS2 inhibitor, potently reduced the abundance of PUFA, including ARA acyl chains of mitochondrial phospholipids, mainly PC and PE. These are the most abundant phospholipids in all cellular membranes including mitochondrial membranes (21, 48-50) and important stores of ARA residues (50-54). FADS2 inhibition also reduced the mitochondrial membrane potential, oxygen consumption and mitochondrial cholesterol uptake and diminished steroidogenesis, which was partially reversed by ARA supplementation. ARA was previously reported to be required for steroidogenesis and high amounts of arachidonic cholesterylesters are found in rat adrenal glands (36, 55). However, this is the first study suggesting that the phospholipidomic landscape and the ARA content of mitochondria specifically determines steroidogenesis. Based on our findings, we present that the PUFA content of phospholipids assembling the mitochondrial membrane is critical for proper mitochondrial membrane potential and bioenergetics, which is pivotal for cholesterol transport into mitochondria and steroidogenesis (14, 23-26).

Abundance of long-chain PUFA in phospholipids constructing phospholipid layers increases membrane fluidity, in contrast to saturated fatty acids, which make membranes more rigid (56, 57). In turn, membrane fluidity can determine enzymatic activity and secretory functions (56-59). Steroid hormones are rapidly synthesized from cholesterol through a cascade of enzymatic reactions taking place in the mitochondria and the ER (60). The activity of the enzymes involved in steroid biosynthesis depends on the membrane fluidity of these organelles (61, 62). Along this line, our data suggest that FADS2 providing PUFA residues may increase membrane fluidity in adrenocortical cells thereby promoting steroidogenesis. In agreement with this hypothesis, FADS2 deficient female and male mice are sterile due to changes in the membrane fluidity of gonadal cells encompassing less PUFA acyl groups (15, 16). Fertility of these mice is restored by dietary supplementation with ARA, EPA and DHA (15, 63). On the other hand, FADS2 deficiency was reported to lead to a non-canonical conversion of linoleate to eicosatrienoic acid (20:3 n-6), which substitutes ARA in phospholipids thereby critically changing the structure and function of ER and Golgi membranes (64). However, the precise mechanisms linking the mitochondrial lipidome with steroidogenesis in the adrenal gland remain to be elucidated.

Having found that the adrenal lipidome can be integral to the regulation of steroidogenesis, we next asked whether it can be modulated in established obesity by FADS2 inhibition. Along this line, we demonstrate that administration of SC-26196 through the food reduced the PUFA content of the adrenal gland and downregulated corticosteroid production in obese mice. We next assessed whether it can be modulated by dietary interventions. To this end, EPA was selected as a candidate dietary component. EPA together with DHA, i.e. the two main fish oil components used for food supplementation, was previously shown to reduce corticosterone levels and anxiety in rats subjected to restrained stress test (65). EPA and DHA supplementation also reduced cortisol saliva levels and perceived stress in abstinent alcoholics (66). Additionally, EPA decreased serum cortisol levels in patients with major depression disorder (67).

Here, we fed mice with HFD for 10 weeks and another 10 weeks with HFD containing or not icosapent ethyl, a highly purified EPA ethyl ester (42). We chose to administrate icosapent ethyl instead of EPA or EPA with DHA, based on the findings of large clinical studies, which showed that icosapent ethyl alone lowered the risk of cardiovascular events, non-HDL cholesterol and triglycerides in patients with established cardiovascular disease, hypertriglyceridemia or diabetes (40-42). We demonstrate that icosapent ethyl food supplementation increased the omega-3 fatty acid content of the adrenal lipidome, instigated transcriptional changes in the adrenal cortex opposite to the ones evoked by HFD, which were associated to lipid metabolism, mitochondrial function and steroid biosynthesis, and reduced corticosterone and aldosterone plasma levels. Accordingly, EPA treatment of cultured adrenocortical cells reduced FADS2 expression and diminished forskolin-stimulated cortisol production, indicating that icosapent ethyl dietary supplementation may directly regulate the steroidogenic function of adrenocortical cells. In agreement with these results, EPA was previously shown to downregulate FADS2 expression in THP-1 cells (68), 3T3-L1 adipocytes (69) and HepG2 hepatocytes (70). Hence, icosapent ethyl is likely to influence the lipidome of not only the adrenal gland but also several other tissues.

Finally, in order to substantiate the role of FADS2 in the human adrenal gland, we analyzed its expression in adrenal non-tumorous and tumorous tissue. We demonstrate that FADS2 expression is higher in aldosterone-producing adenomas compared to non-functioning adenomas or non-tumorous adrenocortical tissue and correlates with the expression of STAR in aldosterone- and cortisol-producing adenomas.

These data collectively demonstrate that FADS2 is a major regulator of steroidogenesis in the adrenal gland due to its key role in shaping the lipidomic landscape of adrenocortical cells. Moreover, our findings indicate that the adrenal lipidome can be modulated by icosapent ethyl dietary supplementation, which consequently influences corticoid production, thereby endorsing the evaluation of icosapent ethyl dietary supplementation as a means of regulation of cortisol and aldosterone levels in obese subjects. Finally, our data indicate a clear correlation between FADS2 expression and steroidogenic capacity in the human adrenal gland and suggest that the role of FADS2-mediated lipidomic changes in adrenocortical tumorigenesis merits further investigation.

### Material and methods:

### Mice and in vivo experiments:

The Fads2^{-/-} mice were developed by Stroud et al (16). The first 4 postnatal weeks female WT and Fads2^{-/-} littermate mice were kept with their mothers and fed a PUFA-rich diet. After weaning at 4 weeks, they were kept for another 10 weeks on a PUFA-rich or low-PUFA diet.

Six weeks-old male WT C57BL/6J mice were fed for 20 weeks a LFD (D12450B, Research Diets, NJ, USA) or a HFD (D12492, Research Diets, NJ, USA). In some experiments, WT C57BL/6J mice were fed for 8 weeks a HFD and for another 8 weeks a HFD supplemented with SC-26196 (Biorbyt Ltd). SC-26196 was mixed in the HFD at a concentration of 625 mg/kg of diet corresponding to approx. 100 mg/kg of animal weight. In other experiments WT mice were fed for 10 weeks a HFD and for another 10 weeks a HFD supplemented with icosapent ethyl (Vascepa). The content of Vascepa capsules was mixed by Research Diets with the diet at a concentration of 1.125 g/kg icosapent ethyl (corresponding to approx. 180 mg/kg of animal weight). Mice were weighed every week. Glucose and insulin tolerance tests were performed as previously described (20, 21). Three to five mg of hair (2-3 mm close to the skin on the back of the mice) were cut while gently holding the mice without anaesthesia.

At the end of the experiment, blood was collected retroorbitally in EDTA tubes, the mice were sacrificed by cervical dislocation and organs were excised. Tissues were immediately snap-frozen or processed for cell isolation. For electron microscopy, mice were anaesthetized with isoflurane and systemically perfused with 4 % paraformaldehyde (PFA), 0.1 % glutaraldehyde (GA), 0.1 M sodium phosphate buffer or PBS (pH 7.4).

Experiments were approved by the Landesdirektion Sachsen, Germany and the institutional animal care and use committee of Azabu University.

### Human samples:

The study included retrospective data and biosamples from 53 patients who underwent surgery because of adrenal tumors, 10 patients with non-functional adenomas, 30 patients with aldosterone-producing adenomas (Conn adenomas), 7 with cortisol-producing adenomas and 6 patients with pheochromocytoma. Patients were enrolled in the ENSAT and the NeoExNet registry studies and the prospective clinical trials, Prospheo/Muppet (https://www.muppet.eu/) and PMT-trial (pmt-study.pressor.org) in three tertiary centers in Germany: University Hospital Carl Gustav Carus, TU Dresden, University Hospital Wuerzburg, and University Hospital Munich, Ludwig-Maximilians-Universität München. All patients provided written informed consent. Measurements of steroids in plasma were performed using LC-MS/MS (71). Potential contamination with medulla tissue was excluded in all samples by examination of tyrosine hydroxylase (TH) and phenylethanolamine N-methyltransferase (PNMT) expression by qPCR. Among the limitations of the study design is that possible influences from antihypertensive medication interfering with the Renin-Angiotensin-Aldosteron-System were not considered in the analysis.

### Sorting of adrenocortical cell populations:

Adrenal glands were isolated and cleaned from the surrounding fat tissue and the adrenal cortex was separated from the medulla under a dissecting microscope. The cortex was collected in a digestion buffer, consisting of collagenase I and bovine serum albumin (both at 1.6 mg/ml concentration, Sigma-Aldrich, Germany) dissolved in PBS, and digested for 25 min at 37 °C while shaking at 900 rpm. Dissociated cells were passed through a 22 G needle and then a 100 µm cell strainer and centrifuged at 300 × g for 5 min at 4 °C. Cells were then washed in MACS buffer (0.5 % BSA, 2 mM EDTA in PBS) and endothelial cells (CD31⁺) and leukocytes (CD45⁺) were sequentially positively selected using CD31 and CD45 MicroBeads (Miltenyi Biotec), respectively, according to manufacturer's instructions. Briefly, pelleted cells were resuspended in 190 µl MACS buffer, mixed with 10 µl CD31 MicroBeads, incubated for 15 min at 4 °C, washed with 2 ml MACS buffer and centrifuged at 300 × g for 10 min. The cell pellet was resuspended in 500 µl MACS buffer and applied onto MS Column placed on MACS Separator. The columns were washed and the flow-through was collected. CD31⁺ cells were positively sorted. The flow-through was centrifuged at 300 × g for 5 min, and the pelleted cells were subjected to the same procedure using CD45 MicroBeads. The flow-through containing CD31-CD45- adrenocortical cells was centrifuged at 300 × g for 5 min, and the pelleted cells were collected. CD45⁺ cells were positively sorted. Collected cell populations were kept for transcriptional analysis.

### Cell and explant culture and treatments:

Adrenal glands or adrenal corteces were isolated from WT C57BL/6J mice, cleaned from the surrounding fat tissue and incubated in collagenase buffer (1.6 mg/ml collagenase type I, 1.6 mg/ml BSA) for 45 min at 37 °C, while shaking at 900 rpm. Dissociated cells were passed through 22 G needle and a 100 µm cell strainer and centrifuged at 300 × g for 5 min at 4 °C. Cells of a pool from two adrenals of each mouse were distributed in four 0.2 % gelatin-precoated wells of a 96-well plate in DMEM/F12 supplemented with 1 % FBS, 50 U/mL penicillin and 50 µg/mL streptomycin. Two hours after seeding 10 µM SC-26196 (Tocris, Wiesbaden-Nordenstadt, Germany) or same amount of carrier (DMSO) were added without changing the medium. In some experiments medium was additionally supplemented with 150 µM ARA (Sigma Aldrich, Germany) or same amount of solvent (endotoxin-free water). When induction of steroidogenesis was required, the medium was changed 18 h after treatment and the cells were incubated with or without 10 ng/mL ACTH for 60 min in the presence of either 10 µM SC-26196 or DMSO. Supernatants were kept for steroid hormone measurement and cell lysates were collected for transcriptional analysis. In some experiments, primary CD31-CD45- adrenocortical cells were treated or not for 18 h with 66 µM EPA (Sigma-Aldrich).

Medullas were isolated from WT C57BL/6J mice and transferred into DMEM/F12 medium. Six hours later the medulla explant supernatants were collected and applied onto adrenocortical cell cultures. These were previously treated for 6 h with SC-26196 (10 µM) or DMSO. SC-26196 or DMSO were re-added and adrenocortical cells were incubated for another 18 h until supernatants were collected for steroid measurement. In other experiments, medulla explants were treated for 6 h with SC-26196 (10 µM) or DMSO, washed thoroughly and left another 18 h in culture. Their supernatant was then applied on adrenocortical cell cultures and 8 h later supernatants of the latter were collected and analyzed by LC-MS/MS.

NCI-H295R cells (obtained from ATCC Cat# CRL-2128) were maintained in DMEM/F12 medium supplemented with 2.5 % Nu-Serum type I (Corning), 1 % Insulin Transferrin Selenium (ITS; Gibco), 50 U/mL penicillin and 50 µg/mL streptomycin. They were treated for 18 h with 10 µM SC-26196 or DMSO. In some experiments, NCI-H295R cells were treated or not for 48 h 66 µM EPA and in the last 24 h they were stimulated or not with forskolin (10 µM; Sigma-Aldrich).

### Cell fractionations:

Cells were trypsinized and pelleted by centrifugation (500xg, 5 min, 4 °C). The cells were gently resuspended in homogenization buffer (200 mM mannitol, 50 mM sucrose, 10 mM KCI, 100 mM 1 mM EDTA 25 mM HEPES, pH 7.4, 1 µM aprotinin, 10 µM leupeptin, 20 µM PMSF) and homogenized on ice with 20 strokes of tight Dounce homogenizer. The samples were then centrifuged (800xg, 10 min, 4 °C) to pellet the nuclei. The supernatant was mixed with iodixanol solution (Optiprep, Sigma-Aldrich) to a final concentration of 20 %. The samples were placed at the bottom of a tube and overlaid with 500 µl iodixanol solutions (18 %, 16 %, 14 %, 12 %, 10 %, 5 %) and finally with 500 µl of HBS (50 mM HEPES, 150 mM NaCl, pH 7.4). The samples were centrifuged in Beckman SW60Ti swing-out rotor (45,000 rpm, 5 h, 4 °C). After centrifugation fifteen 280 µl aliquots were collected from top of the tubes.

Fractions 10-12 (SDHB-positive) and 4-5 (HSL-positive) were considered as mitochondrial and lipid droplet fractions, respectively. Protein concentration was measured in the mitochondrial and lipid droplet pools with the BCA Protein Assay Kit (Thermo Fisher) and fractions were analyzed for cholesterol and CE concentrations.

### Lipidomic analysis of isolated mitochondria:

Fractions containing mitochondria were combined and extracted with three volumes of ice-cold chloroform/methanol (10:1, v/v, both containing 0.1 % butylated hydroxytoluene (Merck, ≥99 %)). Samples were incubated on ice for 15 min, before the organic phase was collected after centrifugation (5 min, 5,000 × g). The aqueous phase was re-extracted with two volumes chloroform/methanol as described above. Combined organic phases were dried in vacuo. Methanol (ULC-MS grade, Biosolve B.V.) and chloroform (EMSURE ACS, ISO, Reag. Ph Eur; Supleco) were of highest purity.

Lipid extracts were dissolved in 50 µL 2-propanol (ULC/MS-CC/SFC grade, >99.95 %; Biosolve B.V.) and analyzed by LC-MS/MS. Lipids (2.5 µL loaded in positive mode, 5 µL in negative mode) were separated by reverse phase chromatography (Accucore C30 column; 150 mm × 2.1 mm 2.6 µM 150 Å, Thermo Fisher Scientific) using a Vanquish Horizon UHPLC system (Thermo Fisher Scientific) coupled on-line to a Q Exactive Plus Hybrid Quadrupole Orbitrap mass spectrometer (Thermo Fisher Scientific, Bremen, Germany) equipped with a HESI source. Column was operated at 50 °C and flow rate of 0.3 mL/min. Chromatographic gradient included following steps: 0-20 min - 10 to 86 % B, 20-22 min - 86 to 95 % B, 22-26 min - 95 % isocratic, 26-26.1 min - 95 to 10 % B, followed by 5 min re-equilibration at 10 % B. Eluent A consisted of acetonitrile/water (50:50, v/v, both ULC/MS-CC/SFC grade, Biosolve B.V.) and Eluent B of 2-propanol/acetonitrile/water (85:10:5, v/v/v), both containing 5 mM ammonium formate (MS grade, Sigma Aldrich) and 0.1 % formic acid (ULC/MS-CC/SFC grade, Biosolve B.V.). HESI parameters: sheath gas - 40 a.u., auxiliary gas - 10 L/min, sweep gas - 1 L/min, spray voltage 3.5 kV (positive ion mode); -2.5 kV (negative ion mode), ion transfer temperature - 300 °C, S-lens RF level - 35 %, aux gas heater temperature - 370°C. Data were acquired using data dependent acquisition (DDA): full scan resolution 140,000 at m/z 200, AGC target 1e6, maximum IT 100 ms, scan range m/z 350-1200; data dependent MS/MS: resolution 17500 at m/z 200, AGC target 1e5, maximum IT 60 ms, loop count 15, isolation window 1.2 m/z, stepped normalized collision energies of 10, 20 and 30 %, AGC target of 2e2, dynamic exclusion 10 s, isotope exclusion on, default charge state - 1) (72).

Lipid identification and relative quantification was performed using Lipostar2 (73). Briefly, supersample filter considering only lipids with isotopic pattern and with an MS/MS spectrum were kept. Features were searched against the LIPID MAPS structural database (downloaded 11/2021), and proposed identifications obtained by automatic approval considering 3 and 4 stars. Data were normalized using the Sum/Average normalization (area sum). Statistical analysis was performed in MetaboAnalyst 5.0 (sample normalization by median, data log transformed, pareto scaled) (74).

### Product ion scan for cholesterol and cholesteryl esters:

For quantification of cholesterol and cholesteryl esters, equal volumes of mitochondria fractions (180 µL) were combined, cholesterol-2,3,4-¹³C (5 nmol, Sigma Aldrich) and SPLASH^{®}Lipidomix^{®} (3 µL, Avanti Polar Lipids Inc) spiked, and samples incubated for 15 min on ice. Lipids were extracted as described above.

Lipid extracts were reconstituted in 55 µL 2-propanol, and 10 µL were loaded onto a Accucore C30 column (150 mm × 2.1 mm 2.6 µM 150 Å, Thermo Fisher Scientific) installed on a Vanquish Horizon UHPLC (Thermo Fisher Scientific) coupled on-line to a Orbitrap Exploris 240 (Thermo Fisher Scientific) mass spectrometer equipped with a HESI source. Lipids were separated at a flow rate of 0.3 mL/min and a column temperature of 50°C by a gradient starting from 10 % B (0-20 min 10 to 80 % B, 20-37 min 80 to 95 % B,37-41 min 95 to 100 % B,41-49 min 100 % B, 49.1-57 min 10 % B). The column was re-equilibrated for 7.9 min at 10 % eluent B. Eluent A consisted of acetonitrile/water (50:50, v/v, both ULC/MS-CC/SFC grade, Biosolve B.V.) and Eluent B of 2-propanol/acetonitrile/water (85:10:5, v/v/v), both containing 5 mM Ammonium formate (MS grade, Sigma Aldrich) and 0.1 % formic acid (ULC/MS-CC/SFC grade, Biosolve B.V.).

For cholesterol and CE, a scheduled product ion scan (PIS) method with the following parameters was used: spray voltage - 3500 V, sheath gas - 40 arb, aux gas - 10 arb, sweep gas - 1 arb, vaporizer - 370°C, ion transfer tube - 300°C, polarity - positive, default charge state - 1, Isolation window - 1 m/z, Resolution at m/z 200 - 45,000, HCD collision energy (%) - 17,27,37, fixed first mass - m/z 70, AGC target - Standard, maximum injection time - auto, EASY-IC - Start run. Quantification was performed in Skyline (Version 22.2.0.255, MacCoss Lab, University of Washington) using cholesten ion (m/z 369.35) as a quantifier (75). CE were quantified relative to the 18:1-d7-cholesteryl ester present in the SPLASH^{®}Lipidomix^{®} mix. Signal of cholesterol-2,3,4-¹³C was used to quantify endogenous cholesterol.

### Shotgun lipidomics and analysis:

Adrenal glands were homogenized in ammonium-bicarbonate buffer (150 mM ammonium bicarbonate, pH 7) with TissueLyser (Qiagen). Protein content was assessed using the BCA Protein Assay Kit (Thermo Fisher). Equivalents of 20 µg of protein were taken for MS analysis. MS-based lipid analysis was performed as described (33, 76). Lipids were extracted using a two-step chloroform (Sigma Aldrich) / methanol (Thermofisher Scientific, Darmstadt, Germany) procedure (77). Samples were spiked with internal lipid standard mixture containing: CL 16:1/15:0/15:0/15:0, Cer 18:1;2/17:0, HexCer 18:1;2/12:0, LPA 17:0, LPC 12:0, LPE 17:1, LPG 17:1, LPI 17:1, LPS 17:1, PA 17:0/17:0, PC 17:0/17:0, PE 17:0/17:0, PG 17:0/17:0, PI 16:0/16:0, PS 17:0/17:0, CE 20:0, SM 18:1;2/12:0;0, cholesterol D6 (all Avanti Polar Lipids), TAG 17:0/17:0/17:0 and DAG 17:0/17:0 (both Larodan Fine Chemicals). Synthetic lipid standards were purchased from Avanti Polar Lipids, Larodan Fine Chemicals and Sigma-Aldrich and all chemicals were analytical grade. After extraction, the organic phase was transferred to an infusion plate and dried in a speed vacuum concentrator (Martin Christ, Germany). The dry extract from the first step was re-suspended in 7.5 mM ammonium acetate (Sigma Aldrich) in chloroform/methanol/propan-2-ol (Thermofisher Scientific) (1:2:4 V:V:V) and the second-step dry extract in 33 % ethanol of methylamine (Sigma Aldrich)/ chloroform/methanol (0.003:5:1 V:V:V) solution. All liquid handling steps were performed using Hamilton Robotics STARlet robotic platform with the Anti Droplet Control feature for organic solvents pipetting.

Samples were analyzed by direct infusion on a QExactive mass spectrometer (Thermo Fisher Scientific) equipped with a TriVersa NanoMate ion source (Advion Biosciences, Ithaca, NY). Samples were analyzed in both positive and negative ion modes with a resolution of R_{(m/z=200)}=28,0000 for MS and R_{(m/z=200)}=17500 for MS/MS experiments, in a single acquisition. MS/MS was triggered by an inclusion list encompassing corresponding MS mass ranges scanned in 1 Da increments (78). Both MS and MS/MS data were combined to monitor CE, DAG and TAG ions as ammonium adducts; PC, PC O-, as acetate adducts; and CL, PA, PE, PE O-, PG, PI and PS as deprotonated anions. MS only was used to monitor LPA, LPE, LPE O-, LPI and LPS as deprotonated anions; Cer, HexCer, SM, LPC and LPC O- as acetate adduct and cholesterol as ammonium adduct of an acetylated derivative (79).

Data were analyzed with an in-house developed lipid identification software based on LipidXplorer (80, 81). Data post-processing and normalization were performed using an in-house developed data management system. Only lipid identifications with a signal-to-noise ratio > 5 and a signal intensity 5-fold higher than in corresponding blank samples were considered for further data analysis. All downstream analyses were performed in R (R Core Team 2019). The dataset was filtered by applying an occupational threshold of 50 %, meaning that lipids that were present in less than 50 % of the samples in a group were set to zero in that group. Dataset was log2-scaled before PCA. The descriptive analysis was performed on the mol %-transformed dataset, i.e. picomol quantities were divided by the sum of the lipids detected in the respective sample and multiplied by 100. Total carbon chain length and double bonds plots result from grouping together all the lipids that present the same number of carbon atoms (total length) or the same number of double bonds (degree of unsaturation) and calculating the mean and standard deviation in each group of samples (LFD and HFD). To further inspect general trends in the degree of unsaturation and length of storage and membrane lipids under different perturbations, the average weighted number of double bonds (Double Bond Index, DBI) and length (Length Index, LI), as previously described (82). Statistical tests were performed on the mol %-transformed data. First, normality was assessed by means of the Shapiro-Wilkinson test, and according to its result either the Welch t-test or the Wilcoxon test was used. P-values were then adjusted following the Benjamini-Hochberg correction. Analysis was done in R (83). Plots were generated using ggplot2 (84).

### Lipid isomer and oxylipin measurement:

Frozen adrenal glands were homogenized in 200 µL PBS-buffer. Lipids were extracted from adrenal lysates or supernatants in chloroform/methanol (Folch's protocol). The extract was evaporated to dryness under stream of nitrogen at 40 °C. The residue was dissolved in 100 µL ethanol. For FFA 50 µL were used. HPLC-measurement of FFA was performed using an Agilent 1290 HPLC system with binary pump, autosampler and column thermostat equipped with a Phenomenex Kinetex-C18 column 2.6 µm, 2.1 × 150 mm column (Phenomenex, Aschaffenburg, DE) using a solvent system of acetic acid (0.05 %) and acetonitrile. All solvents and buffers in LC-MS-grade were purchased from VWR Germany. The HPLC was coupled with an Agilent 6470 triplequad mass spectrometer with electrospray ionisation source operated in negative multiple reaction monitoring (MRM). The quantification was performed with individual calibration curves using deuterated standards (Cayman Chemical, Ann Arbor, MI).

To release of fatty acids and lipid mediators from phospholipids, 50 µL lipid extract were dissolved with 500 µL Phospholipase A2 (PLA2)-buffer consisting of 80 mM HEPES, 300 mM NaCl, 20 mM CaCl₂ and 2 mg/mL BSA in 60 % glycerol, pH 7.4. This solution was spiked with 5 µL butylhydroxytoluen and 2.2 Units / 26 µL PLA2 from honey bee (Merck, Darmstadt, DE) and incubated one hour at 25 °C. Hundred µL of hydrolysate were analyzed as described above. The results were the sum of free fatty acids and fatty acids from phospholipids, hence the amount of fatty acyl chains of phospholipids was calculated by subtracting the amount of FFA from phospholipidic fatty acyl chains.

For measurement of lipid mediators 400 µL of hydrolysate were spiked with 100 pg d4-PGE2-13 as an internal standard (Cayman Chemical, Ann Arbor, USA). Acetonitrile (400 µl) was added for protein precipitation. After centrifugation and pH adjustment at 6.0, the obtained supernatant was added to Bond Elute Certify II columns (Agilent Technologies, Santa Clara, USA) for Solid phase Extraction. The eluate was evaporated on a heating block at 40 °C under a stream of nitrogen to obtain solid residues, which were dissolved in 100 µL methanol/water (60/40). The residues were analysed using an Agilent 1290 HPLC system with binary pump, multisampler and column thermostat with a Zorbax Eclipse plus C-18, 2.1 × 150 mm, 1.8 µm column using a gradient solvent system of aqueous acetic acid (0.05 %) and acetonitrile / methanol 50:50. The flow rate was set at 0.3 mL/min, the injection volume was 20 µL. The HPLC was coupled with an Agilent 6495 Triplequad mass spectrometer (Agilent Technologies, Santa Clara, USA) with electrospray ionisation source. Analysis was performed with Multiple Reaction Monitoring in negative mode, at least two mass transitions for each compound.

### Steroid hormone measurement:

Steroid hormones in plasma and cell culture supernatants were analyzed by LC-MS/MS as previously described (71). Fifty to hundred µL plasma or cell culture supernatants were extracted by solid phase extraction using positive pressure, followed by a dry-down under gentle stream of nitrogen. Residues were reconstituted in 100 µL of the initial LC mobile phase and 10 µL were injected for detection by the triple quadrupole mass spectrometer in multiple reaction-monitoring scan mode using positive electrospray ionization. Quantification of steroid concentrations was done by comparisons of ratios of analyte peak areas to respective peak areas of stable isotope labeled internal standards obtained in samples to those of calibrators.

Corticosterone and 11-dehydrocorticosterone in hair were extracted with methanol as described elsewhere. In brief, hair (3-5 mg) was washed by addition of 2.5 mL isopropanol. After 3 minutes the isopropanol was decanted and hair samples were dried in a fume hood. After drying, hair samples were extracted in 1.8 mL methanol that contained the internal standard mixtures for 18 h at room temperature. Finally, the methanolic extract was dried down, followed by reconstitution in respective mobile phase and analysis by LC-MS/MS as previously described with minor modifications. In brief, a QTRAP 6500+ (Sciex, Darmstadt) coupled to an Aquity i-class ultra-performance liquid chromatography system (Waters, Eschborn) was used. Chromatographic separation was achieved as described before. Corticosterone and 11-dehydrocorticosterone were detected by using the multi reaction monitoring scan mode with respective quantifier ions of 347.1->329.2 and 345.1->121.1, and qualifier ions of 347.1->90.9 and 345.1->242.3. An automatic flow injection analysis revealed optimal ion source settings of curtain gas (40), ion spray voltage (5500), temperature (500), gas 1 (70) and gas 2 (50). Quantification of steroid hormones was achieved by comparisons of ratios of analyte peak areas to internal standard peak areas observed in samples to those in calibrators. Measured steroid concentrations were normalized to sample weights.

### ACTH measurement:

ACTH was measured in EDTA mouse plasma 4x diluted in PBS with an ELISA kit (Abnova, KA0917) according to manufacturer's instructions.

### RNA-Seq:

For transcriptome mapping, strand-specific paired-end sequencing libraries from total RNA were constructed using TruSeq stranded Total RNA kit (Illumina Inc). Sequencing was performed on an Illumina HiSeq3000 (1x75 basepairs). Low quality nucleotides were removed with the Illumina fastq filter and reads were further subjected to adaptor trimming using cutadapt (85). Alignment of the reads to the Mouse genome was done using STAR Aligner (86) using the parameters: "-runMode alignReads -outSAMstrandField intronMotif-outSAMtype BAM SortedByCoordinate-readFilesCommand zcat". Mouse Genome version GRCm38 (release M12 GENCODE) was used for the alignment. The parameters: `htseq-count -f bam -s reverse -m union -a 20', HTSeq-0.6.1p1 (87) were used to count the reads that map to the genes in the aligned sample files. The GTF file (gencode.vM12.annotation.gtf) used for read quantification was downloaded from Gencode (https://www.gencodegenes.org/mouse/release_M12.html). Gene centric differential expression analysis was performed using DESeq2_1.8.1 (88). The raw read counts for the genes across the samples were normalized using 'rlog' command of DESeq2 and subsequently these values were used to render a PCA plot using ggplot2_1.0.1 (89).

Pathway and functional analyses were performed using GSEA (89) and EGSEA (90). GSEA is a stand-alone software with a GUI. To run GSEA, a ranked list of all the genes from DESeq2 based calculations was created by taking the -log10 of the p-value and multiplying it with the sign the of the fold change. This ranked list was then queried against Molecular Signatures Database (MsigDB), KEGG, GO, Reactome and Hallmark based repositories. EGSEA is an R/Bioconductor based command-line package. For doing functional analyses using EGSEA a differentially expressed list of genes with parameters log2foldchange > 0.3 and padj < 0.05 was used. The KEGG database repository was used for performing the functional analyses.

For constructing heatmaps, the "rlog-normalized" expression values of the significantly expressed genes (padj < 0.05) was scaled using z-transformation. The resulting matrices were visually rendered using MORPHEUS.

### Electron microscopy:

After animal perfusion with PFA/GA, adrenal glands were immediately transferred into 4 % PFA, 2 % GA, 0.1 M sodium phosphate buffer or PBS (pH 7.4) for 1 h at room temperature (RT), and stored in 0.1 % GA 1 % PFA in PBS at 4 °C until processing. After washing with PBS, adrenal glands were further dissected into 3 pieces. They were contrasted with 1 % osmium tetroxide/1.5 % potassium cyanoferrate for an hour at RT. Contrast was further enhanced by incubation with 0.5 % uranyl acetate overnight in the cold. Dehydration with a graded series of ethanol followed (10 min each step, 3 steps of pure ethanol for 20 min). Samples were gradually infiltrated and embedded in Epon resin and cured at 60 °C for 48 h. Ultrathin sections (70 nm) were cut on a Leica UC7 ultramicrotome and post-stained with uranyl and lead. Samples were evaluated and images acquired on a Tecnai Biotwin T12 transmission electron microscope (Philips/FEI/ Thermofisher Scientific) with a digital F416 CCD camera (TVIPS).

### Quantification of mitochondrial structural features:

Mitochondrial features (area, perimeter, circularity and aspect ratio) were analyzed in electron microscope images using the Fiji software (91) and the 'particle analysis' plugin. Outlines of mitochondria were marked by hand using the `freehand selection' tool. At least 16 images obtained from different areas were analyzed per adrenal gland.

### Transcriptional analysis:

RNA was isolated from frozen tissues with the TRI Reagent (MRC, Cincinnati, USA) after mechanical tissue disruption or from cells with the RNeasy Mini Kit (Qiagen, Düsseldorf, Germany) according to manufacturer's instructions. RNA obtained with Tri Reagent was subsequently extracted with Chloroform and the NucleoSpin RNA, Maxi kit (Macherey-Nagel, Düren, Germany). Reverse transcription was performed with the iScript cDNA Synthesis kit (Biorad, Munich, Germany) and cDNA was analyzed by qPCR using the SsoFast Eva Green Supermix (Bio-Rad, Munich, Germany), a CFX384 real-time System C1000 Thermal Cycler (Bio-Rad) and the Bio-Rad CFX Manager 3.1 software. Primers used are listed in Table S6.

### Plasmid transfection

NCI-H295R cells were transfected with a FADS2-overexpressing plasmid (Origene NM_004265) or control pCMV plasmid. Briefly, cells were plated in 24-well plates at 70% confluence and the next day they were transfected with 0.3 µg DNA using Lipofectamine LTX with Plus Reagent (ThermoFisher Scientific), according to manufacturer's instructions.

### Western blot analysis:

Cells or tissues were lysed with 10 mM Tris-HCl, pH7.4 + 1 % SDS + 1 mM sodium vanadate, cell lysates were centrifuged at 16,000 g for 5 min at 4 °C and supernatants were collected. Total protein concentration was measured using the BCA Protein Assay Kit (Thermo Scientific) in cell lysates or cell fractions. Protein samples were prepared with 5x Reducing Laemmli buffer containing same amount of protein, denatured at 95 °C for 5 min and loaded on a 10 % acrylamide gel (Invitrogen) for sodium dodecyl sulfate polyacrylamide gel electrophoresis (SDS-PAGE). PageRuler Prestained Protein Ladder (Thermo Fisher Scientific) was used as a protein size ladder. The separated proteins were transferred on Amersham Protran nitrocellulose membrane (GE Healthcare Lifescience). After blocking with 5 % skimmed milk in TBS-T (0.1 % Tween-20 (Sigma-Aldrich) in 1x Tris-buffered saline) for 1 h at RT, membranes were incubated overnight at 4 °C with anti-FADS2 (1:2,000; Sigma Aldrich, #PA-87765), anti-StAR (1:300; Santa Cruz, #SC-25806), anti-SDHB (1:1,000; Sigma-Aldrich, #HPA002868), anti-HSL (1:1,000, Cell Signaling, #4107 ), anti-Tubulin (1:3,000; Sigma-Aldrich, T5186) or anti-Vinculin (1:3,000; Cell Signaling #4650), diluted in 5 % BSA in TBS-T. After washing, membranes were incubated for 1 h at RT with secondary antibodies: goat anti-rabbit IgG HRP- conjugated (1:3,000; Jackson ImmunoResearch) or goat anti-mouse IgG HRP-conjugated (1:3,000; Jackson ImmunoResearch), diluted in 5 % skimmed milk in TBS-T. The signal was detected using the Western Blot Ultra-Sensitive HRP Substrate (Takara) and imaged using the Fusion FX Imaging system (PeqLab Biotechnologie). Immunofluorescence:
Adrenal glands cleaned from surrounding fat tissue were fixed in 4 % PFA in PBS, washed overnight in PBS, cryopreserved in 30 % sucrose (AppliChem GmbH) in PBS overnight at 4°C, embedded in O.C.T. Compound (Tissue-Tek) and frozen at -80 °C. Each adrenal gland was cut into 8 µm thick serial sections. Before staining, adrenal sections were pre-warmed at RT for 30 min. Adrenal sections were washed twice with PBS, permeabilized with 0.5 % Triton X-100 in PBS for 15 min, treated with TrueBlack Lipofuscin Quencher (1:40 in 70 % ethanol; Biotium) for 30 sec to reduce autofluorescence and blocked in Dako Protein Block, serum-free buffer for 1 h at RT. Then, sections were incubated overnight at 4 °C with anti-FADS2 (1:250, Sigma Aldrich, #PA-87765), washed with PBS, and incubated for 2 h at RT with the secondary antibody Alexa Fluor 555 donkey anti-rabbit (1:300; Thermo fisher Scientific, #A- 31572) together with DAPI (1:5,000; Roche), both diluted in Dako Antibody Diluent. After washing with PBS, cryosections were mounted with Fluoromount (Sigma-Aldrich), covered with 0.17 mm cover glass, fixed with nail polish and kept at 4 °C until imaging. Adrenal cross sections were imaged as tile sections taken with an Axio Observer.Z1 (Zeiss) and a Plan-Apochromat 20x objective.

### Histology:

Adrenal glands were fixed in 4 % PFA and paraffin embedded. Sections were stained with hematoxylin and eosin. Images were obtained using an Axio Observer.Z1 (Zeiss) and a Plan-Apochromat 20x objective.

### Flow cytometry:

Adrenocortical cells (CD31-CD45-) were treated for 18 h with DMSO or SC-26196 (10 µM) and were then incubated with Mitotracker Green (0.25 µM; Invitrogen), TMRE (2.5 µM; Thermofisher), for 30 min at 37°C in FACS buffer (0.5 % BSA, 2 mM EDTA in PBS) in dark. Live cells were selected by DAPI staining. FACS was performed using LSR Fortessa X20 flow cytometer and data were analyzed with the FlowJo software.

### Seahorse analysis:

Primary adrenocortical cells were plated in 0.2 % gelatin-precoated XF96 cell culture microplate (Agilent) (8*10⁴ cells/well) and treated for 18 h with DMSO or SC-26196 (10 µM). OCR was measured with a Seahorse XF96 Analyzer (Agilent Technologies). The experimental medium used was XF Base Medium supplemented with glucose (10 mM), pyruvate (1 mM) and glutamine (2 mM).

### Statistical analysis:

Data were analyzed with Mann-Whitney U test, Students t-test, one-way ANOVA with post-hoc Tukey's test for multiple comparisons or Pearson analysis with p<0.05 set as a significance level using the GraphPrism 7 software.

### References

1. R. D. Feldman, Aldosterone and blood pressure regulation: recent milestones on the long and winding road from electrocortin to KCNJ5, GPER, and beyond. Hypertension 63, 19-21 (2014).
2. S. L. Lightman, M. T. Birnie, B. L. Conway-Campbell, Dynamics of ACTH and Cortisol Secretion and Implications for Disease. Endocr Rev 41, (2020).
3. L. Magomedova, C. L. Cummins, Glucocorticoids and Metabolic Control. Handb Exp Pharmacol 233, 73-93 (2016).
4. L. S. Santana, A. G. Guimaraes, M. Q. Almeida, Pathogenesis of Primary Aldosteronism: Impact on Clinical Outcome. Front Endocrinol (Lausanne) 13, 927669 (2022).
5. R. Pivonello, A. M. Isidori, M. C. De Martino, J. Newell-Price, B. M. Biller, A. Colao, Complications of Cushing's syndrome: state of the art. Lancet Diabetes Endocrinol 4, 611-629 (2016).
6. D. A. Calhoun, K. Sharma, The role of aldosteronism in causing obesity-related cardiovascular risk. Cardiol Clin 28, 517-527 (2010).
7. M. M. Swierczynska, I. Mateska, M. Peitzsch, S. R. Bornstein, T. Chavakis, G. Eisenhofer, V. Lamounier-Zepter, S. Eaton, Changes in morphology and function of adrenal cortex in mice fed a high-fat diet. Int J Obes (Lond) 39, 321-330 (2015).
8. A. Hofmann, M. Peitzsch, C. Brunssen, J. Mittag, A. Jannasch, A. Frenzel, N. Brown, S. M. Weldon, G. Eisenhofer, S. R. Bornstein, H. Morawietz, Elevated Steroid Hormone Production in the db/db Mouse Model of Obesity and Type 2 Diabetes. Horm Metab Res 49, 43-49 (2017).
9. J. Galitzky, A. Bouloumie, Human visceral-fat-specific glucocorticoid tuning of adipogenesis. Cell Metab 18, 3-5 (2013).
10. C. Roberge, A. C. Carpentier, M. F. Langlois, J. P. Baillargeon, J. L. Ardilouze, P. Maheux, N. Gallo-Payet, Adrenocortical dysregulation as a major player in insulin resistance and onset of obesity. Am J Physiol Endocrinol Metab 293, E1465-1478 (2007).
11. P. Q. Barrett, N. A. Guagliardo, P. M. Klein, C. Hu, D. T. Breault, M. P. Beenhakker, Role of voltage-gated calcium channels in the regulation of aldosterone production from zona glomerulosa cells of the adrenal cortex. J Physiol 594, 5851-5860 (2016).
12. W. Kanczkowski, A. Chatzigeorgiou, M. Samus, N. Tran, K. Zacharowski, T. Chavakis, S. R. Bornstein, Characterization of the LPS-induced inflammation of the adrenal gland in mice. Mol Cell Endocrinol 371, 228-235 (2013).
13. S. L. Lightman, M. T. Birnie, B. L. Conway-Campbell, Dynamics of ACTH and cortisol secretion and implications for disease. Endocr Rev, (2020).
14. A. Midzak, V. Papadopoulos, Adrenal Mitochondria and Steroidogenesis: From Individual Proteins to Functional Protein Assemblies. Front Endocrinol (Lausanne) 7, 106 (2016).
15. W. Stoffel, B. Holz, B. Jenke, E. Binczek, R. H. Gunter, C. Kiss, I. Karakesisoglou, M. Thevis, A. A. Weber, S. Arnhold, K. Addicks, Delta6-desaturase (FADS2) deficiency unveils the role of omega3- and omega6-polyunsaturated fatty acids. EMBO J 27, 2281-2292 (2008).
16. C. K. Stroud, T. Y. Nara, M. Roqueta-Rivera, E. C. Radlowski, P. Lawrence, Y. Zhang, B. H. Cho, M. Segre, R. A. Hess, J. T. Brenna, W. M. Haschek, M. T. Nakamura, Disruption of FADS2 gene in mice impairs male reproduction and causes dermal and intestinal ulceration. J Lipid Res 50, 1870-1880 (2009).
17. L. F. Castro, D. R. Tocher, O. Monroig, Long-chain polyunsaturated fatty acid biosynthesis in chordates: Insights into the evolution of Fads and Elovl gene repertoire. Prog Lipid Res 62, 25-40 (2016).
18. W. J. Park, K. S. Kothapalli, P. Lawrence, C. Tyburczy, J. T. Brenna, An alternate pathway to long-chain polyunsaturates: the FADS2 gene product Delta8-desaturates 20:2n-6 and 20:3n-3. J Lipid Res 50, 1195-1202 (2009).
19. B. Y. Lau, D. J. Cohen, W. E. Ward, D. W. Ma, Investigating the role of polyunsaturated fatty acids in bone development using animal models. Molecules 18, 14203-14227 (2013).
20. G. Paradies, V. Paradies, F. M. Ruggiero, G. Petrosillo, Role of Cardiolipin in Mitochondrial Function and Dynamics in Health and Disease: Molecular and Pharmacological Aspects. Cells 8, (2019).
21. J. E. Vance, Historical perspective: phosphatidylserine and phosphatidylethanolamine from the 1800s to the present. J Lipid Res 59, 923-944 (2018).
22. A. Poulaki, S. Giannouli, Mitochondrial Lipids: From Membrane Organization to Apoptotic Facilitation. Int J Mol Sci 23, (2022).
23. S. R. King, Z. Liu, J. Soh, S. Eimerl, J. Orly, D. M. Stocco, Effects of disruption of the mitochondrial electrochemical gradient on steroidogenesis and the Steroidogenic Acute Regulatory (StAR) protein. J Steroid Biochem Mol Biol 69, 143-154 (1999).
24. D. B. Hales, J. A. Allen, T. Shankara, P. Janus, S. Buck, T. Diemer, K. H. Hales, Mitochondrial function in Leydig cell steroidogenesis. Ann N Y Acad Sci 1061, 120-134 (2005).
25. I. Mateska, A. Witt, E. Haga, A. Sinha, C. Yilmaz, E. Thanou, N. Sun, O. Kolliniati, M. Patschin, H. Abdelmegeed, H. Henneicke, W. Kanczkowski, B. Wielockx, C. Tsatsanis, A. Dahl, A. Walch, K. Li, M. Peitzsch, T. Chavakis, V. Alexaki, Inflammation-induced downregulation of succinate dehydrogenase regulates adrenocortical function. BioRxiv, (2022).
26. L. Issop, M. B. Rone, V. Papadopoulos, Organelle plasticity and interactions in cholesterol transport and steroid biosynthesis. Mol Cell Endocrinol 371, 34-46 (2013).
27. R. Garcia-Martin, V. I. Alexaki, N. Qin, M. F. Rubin de Celis, M. Economopoulou, A. Ziogas, B. Gercken, K. Kotlabova, J. Phieler, M. Ehrhart-Bornstein, S. R. Bornstein, G. Eisenhofer, G. Breier, M. Bluher, J. Hampe, A. El-Armouche, A. Chatzigeorgiou, K. J. Chung, T. Chavakis, ADIPOCYTE-SPECIFIC HIF2alpha DEFICIENCY EXACERBATES OBESITY-INDUCED BROWN ADIPOSE TISSUE DYSFUNCTION AND METABOLIC DYSREGULATION. Mol Cell Biol, (2015).
28. K. J. Chung, A. Chatzigeorgiou, M. Economopoulou, R. Garcia-Martin, V. I. Alexaki, I. Mitroulis, M. Nati, J. Gebler, T. Ziemssen, S. E. Goelz, J. Phieler, J. H. Lim, K. P. Karalis, T. Papayannopoulou, M. Bluher, G. Hajishengallis, T. Chavakis, A self-sustained loop of inflammation-driven inhibition of beige adipogenesis in obesity. Nat Immunol 18, 654-664 (2017).
29. Z. Michailidou, M. Gomez-Salazar, V. I. Alexaki, Innate Immune Cells in the Adipose Tissue in Health and Metabolic Disease. J Innate Immun 14, 4-30 (2022).
30. A. Chatzigeorgiou, K. J. Chung, R. Garcia-Martin, V. I. Alexaki, A. Klotzsche-von Ameln, J. Phieler, D. Sprott, W. Kanczkowski, T. Tzanavari, M. Bdeir, S. Bergmann, M. Cartellieri, M. Bachmann, P. Nikolakopoulou, A. Androutsellis-Theotokis, G. Siegert, S. R. Bornstein, M. H. Muders, L. Boon, K. P. Karalis, E. Lutgens, T. Chavakis, Dual role of B7 costimulation in obesity-related nonalcoholic steatohepatitis and metabolic dysregulation. Hepatology 60, 1196-1210 (2014).
31. V. I. Alexaki, The Impact of Obesity on Microglial Function: Immune, Metabolic and Endocrine Perspectives. Cells 10, (2021).
32. S. Dionysopoulou, E. Charmandari, A. Bargiota, N. Vlahos, G. Mastorakos, G. Valsamakis, The Role of Hypothalamic Inflammation in Diet-Induced Obesity and Its Association with Cognitive and Mood Disorders. Nutrients 13, (2021).
33. M. Grzybek, A. Palladini, V. I. Alexaki, M. A. Surma, K. Simons, T. Chavakis, C. Klose, U. Coskun, Comprehensive and quantitative analysis of white and brown adipose tissue by shotgun lipidomics. Mol Metab 22, 12-20 (2019).
34. M. R. Pergande, F. Serna-Perez, S. B. Mohsin, J. Hanek, S. M. Cologna, Lipidomic Analysis Reveals Altered Fatty Acid Metabolism in the Liver of the Symptomatic Niemann-Pick, Type C1 Mouse Model. Proteomics 19, e1800285 (2019).
35. X. Wang, L. P. Walsh, A. J. Reinhart, D. M. Stocco, The role of arachidonic acid in steroidogenesis and steroidogenic acute regulatory (StAR) gene and protein expression. J Biol Chem 275, 20204-20209 (2000).
36. A. Duarte, A. F. Castillo, R. Castilla, P. Maloberti, C. Paz, E. J. Podesta, F. Cornejo Maciel, An arachidonic acid generation/export system involved in the regulation of cholesterol transport in mitochondria of steroidogenic cells. FEBS Lett 581, 4023-4028 (2007).
37. R. A. Frye, R. W. Holz, The relationship between arachidonic acid release and catecholamine secretion from cultured bovine adrenal chromaffin cells. J Neurochem 43, 146-150 (1984).
38. R. K. Saini, Y. S. Keum, Omega-3 and omega-6 polyunsaturated fatty acids: Dietary sources, metabolism, and significance - A review. Life Sci 203, 255-267 (2018).
39. K. Kaliannan, X. Y. Li, B. Wang, Q. Pan, C. Y. Chen, L. Hao, S. Xie, J. X. Kang, Multi-omic analysis in transgenic mice implicates omega-6/omega-3 fatty acid imbalance as a risk factor for chronic disease. Commun Biol 2, 276 (2019).
40. H. E. Bays, C. M. Ballantyne, J. J. Kastelein, J. L. Isaacsohn, R. A. Braeckman, P. N. Soni, Eicosapentaenoic acid ethyl ester (AMR101) therapy in patients with very high triglyceride levels (from the Multi-center, plAcebo-controlled, Randomized, double-blINd, 12-week study with an open-label Extension [MARINE] trial). Am J Cardiol 108, 682-690 (2011).
41. C. M. Ballantyne, H. E. Bays, J. J. Kastelein, E. Stein, J. L. Isaacsohn, R. A. Braeckman, P. N. Soni, Efficacy and safety of eicosapentaenoic acid ethyl ester (AMR101) therapy in statin-treated patients with persistent high triglycerides (from the ANCHOR study). Am J Cardiol 110, 984-992 (2012).
42. D. L. Bhatt, P. G. Steg, M. Miller, E. A. Brinton, T. A. Jacobson, S. B. Ketchum, R. T. Doyle, Jr., R. A. Juliano, L. Jiao, C. Granowitz, J. C. Tardif, C. M. Ballantyne, R.-I. Investigators, Cardiovascular Risk Reduction with Icosapent Ethyl for Hypertriglyceridemia. N Engl J Med 380, 11-22 (2019).
43. Z. Tang, B. Kang, C. Li, T. Chen, Z. Zhang, GEPIA2: an enhanced web server for large-scale expression profiling and interactive analysis. Nucleic Acids Res 47, W556-W560 (2019).
44. M. Bose, R. M. Whittal, W. L. Miller, H. S. Bose, Steroidogenic activity of StAR requires contact with mitochondrial VDAC1 and phosphate carrier protein. J Biol Chem 283, 8837-8845 (2008).
45. N. Strushkevich, F. MacKenzie, T. Cherkesova, I. Grabovec, S. Usanov, H. W. Park, Structural basis for pregnenolone biosynthesis by the mitochondrial monooxygenase system. Proc Natl Acad Sci USA 108, 10139-10143 (2011).
46. A. S. Berthon, E. Szarek, C. A. Stratakis, PRKACA: the catalytic subunit of protein kinase A and adrenocortical tumors. Front Cell Dev Biol 3, 26 (2015).
47. B. P. Schimmer, P. C. White, Minireview: steroidogenic factor 1: its roles in differentiation, development, and disease. Mol Endocrinol 24, 1322-1337 (2010).
48. D. Patel, S. N. Witt, Ethanolamine and Phosphatidylethanolamine: Partners in Health and Disease. Oxid Med Cell Longer 2017, 4829180 (2017).
49. J. N. van der Veen, J. P. Kennelly, S. Wan, J. E. Vance, D. E. Vance, R. L. Jacobs, The critical role of phosphatidylcholine and phosphatidylethanolamine metabolism in health and disease. Biochim Biophys Acta Biomembr 1859, 1558-1572 (2017).
50. S. Furse, A. I. de Kroon, Phosphatidylcholine's functions beyond that of a membrane brick. Mol Membr Biol 32, 117-119 (2015).
51. H. Cadas, E. di Tomaso, D. Piomelli, Occurrence and biosynthesis of endogenous cannabinoid precursor, N-arachidonoyl phosphatidylethanolamine, in rat brain. J Neurosci 17, 1226-1242 (1997).
52. A. R. Brash, Arachidonic acid as a bioactive molecule. J Clin Invest 107, 1339-1345 (2001).
53. M. K. Montgomery, S. H. J. Brown, T. W. Mitchell, A. C. F. Coster, G. J. Cooney, N. Turner, Association of muscle lipidomic profile with high-fat diet-induced insulin resistance across five mouse strains. Sci Rep 7, 13914 (2017).
54. T. Hashidate-Yoshida, T. Harayama, D. Hishikawa, R. Morimoto, F. Hamano, S. M. Tokuoka, M. Eto, M. Tamura-Nakano, R. Yanobu-Takanashi, Y. Mukumoto, H. Kiyonari, T. Okamura, Y. Kita, H. Shindou, T. Shimizu, Fatty acid remodeling by LPCAT3 enriches arachidonate in phospholipid membranes and regulates triglyceride transport. Elife 4, (2015).
55. B. Cheng, J. Kowal, Analysis of adrenal cholesteryl esters by reversed phase high performance liquid chromatography. J Lipid Res 35, 1115-1121 (1994).
56. W. Fan, R. M. Evans, Turning up the heat on membrane fluidity. Cell 161, 962-963 (2015).
57. K. Hac-Wydro, P. Wydro, The influence of fatty acids on model cholesterol/phospholipid membranes. Chem Phys Lipids 150, 66-81 (2007).
58. H. Sunshine, M. L. Iruela-Arispe, Membrane lipids and cell signaling. Curr Opin Lipidol 28, 408-413 (2017).
59. J. C. Holthuis, A. K. Menon, Lipid landscapes and pipelines in membrane homeostasis. Nature 510, 48-57 (2014).
60. F. B. Kraemer, Adrenal cholesterol utilization. Mol Cell Endocrinol 265-266, 42-45 (2007).
61. J. Gallay, M. Vincent, C. de Paillerets, M. Rogard, A. Alfsen, Relationship between the activity of the 3 beta-hydroxysteroid dehydrogenase from bovine adrenal cortex microsomes and membrane structure. Influence of proteins and steroid substrates on lipid "microviscosity". J Biol Chem 256, 1235-1241 (1981).
62. S. Narasimhulu, Thermotropic transitions in fluidity of bovine adrenocortical microsomal membrane and substrate-cytochrome P-450 binding reaction. Biochim Biophys Acta 487, 378-387 (1977).
63. M. Roqueta-Rivera, C. K. Stroud, W. M. Haschek, S. J. Akare, M. Segre, R. S. Brush, M. P. Agbaga, R. E. Anderson, R. A. Hess, M. T. Nakamura, Docosahexaenoic acid supplementation fully restores fertility and spermatogenesis in male delta-6 desaturase-null mice. J Lipid Res 51, 360-367 (2010).
64. W. Stoffel, I. Hammels, B. Jenke, E. Binczek, I. Schmidt-Soltau, S. Brodesser, M. Odenthal, M. Thevis, Obesity resistance and deregulation of lipogenesis in Delta6-fatty acid desaturase (FADS2) deficiency. EMBO Rep 15, 110-120 (2014).
65. M. A. Perez, G. Terreros, A. Dagnino-Subiabre, Long-term omega-3 fatty acid supplementation induces anti-stress effects and improves learning in rats. Behav Brain Funct 9, 25 (2013).
66. P. Barbadoro, I. Annino, E. Ponzio, R. M. Romanelli, M. M. D'Errico, E. Prospero, A. Minelli, Fish oil supplementation reduces cortisol basal levels and perceived stress: a randomized, placebo-controlled trial in abstinent alcoholics. Mol Nutr Food Res 57, 1110-1114 (2013).
67. S. Jazayeri, S. A. Keshavarz, M. Tehrani-Doost, M. Djalali, M. Hosseini, H. Amini, M. Chamari, A. Djazayery, Effects of eicosapentaenoic acid and fluoxetine on plasma cortisol, serum interleukin-1beta and interleukin-6 concentrations in patients with major depressive disorder. Psychiatry Res 178, 112-115 (2010).
68. P. J. Gillies, S. K. Bhatia, L. A. Belcher, D. B. Hannon, J. T. Thompson, J. P. Vanden Heuvel, Regulation of inflammatory and lipid metabolism genes by eicosapentaenoic acid-rich oil. J Lipid Res 53, 1679-1689 (2012).
69. J. C. Ralston, S. Matravadia, N. Gaudio, G. P. Holloway, D. M. Mutch, Polyunsaturated fatty acid regulation of adipocyte FADS1 and FADS2 expression and function. Obesity (Silver Spring) 23, 725-728 (2015).
70. S. Tanaka, N. Ishihara, S. Suzuki, Y. Watanabe, D. Nagayama, T. Yamaguchi, M. Ohira, A. Saiki, T. Tanaka, I. Tatsuno, Fatty acid desaturase 2 is up-regulated by the treatment with statin through geranylgeranyl pyrophosphate-dependent Rho kinase pathway in HepG2 cells. Sci Rep 9, 10009 (2019).
71. M. Peitzsch, T. Dekkers, M. Haase, F. C. Sweep, I. Quack, G. Antoch, G. Siegert, J. W. Lenders, J. Deinum, H. S. Willenberg, G. Eisenhofer, An LC-MS/MS method for steroid profiling during adrenal venous sampling for investigation of primary aldosteronism. J Steroid Biochem Mol Biol 145, 75-84 (2015).
72. M. Lange, G. Angelidou, Z. Ni, A. Criscuolo, J. Schiller, M. Bluher, M. Fedorova, AdipoAtlas: A reference lipidome for human white adipose tissue. Cell Rep Med 2, 100407 (2021).
73. L. Goracci, S. Tortorella, P. Tiberi, R. M. Pellegrino, A. Di Veroli, A. Valeri, G. Cruciani, Lipostar, a Comprehensive Platform-Neutral Cheminformatics Tool for Lipidomics. Anal Chem 89, 6257-6264 (2017).
74. Z. Pang, G. Zhou, J. Ewald, L. Chang, O. Hacariz, N. Basu, J. Xia, Using MetaboAnalyst 5.0 for LC-HRMS spectra processing, multi-omics integration and covariate adjustment of global metabolomics data. Nat Protoc 17, 1735-1761 (2022).
75. K. J. Adams, B. Pratt, N. Bose, L. G. Dubois, L. St John-Williams, K. M. Perrott, K. Ky, P. Kapahi, V. Sharma, M. J. MacCoss, M. A. Moseley, C. A. Colton, B. X. MacLean, B. Schilling, J. W. Thompson, C. Alzheimer's Disease Metabolomics, Skyline for Small Molecules: A Unifying Software Package for Quantitative Metabolomics. J Proteome Res 19, 1447-1458 (2020).
76. J. L. Sampaio, M. J. Gerl, C. Klose, C. S. Ejsing, H. Beug, K. Simons, A. Shevchenko, Membrane lipidome of an epithelial cell line. Proc Natl Acad Sci U S A 108, 1903-1907 (2011).
77. C. S. Ejsing, J. L. Sampaio, V. Surendranath, E. Duchoslav, K. Ekroos, R. W. Klemm, K. Simons, A. Shevchenko, Global analysis of the yeast lipidome by quantitative shotgun mass spectrometry. Proc Natl Acad Sci U S A 106, 2136-2141 (2009).
78. M. A. Surma, R. Herzog, A. Vasilj, C. Klose, N. Christinat, D. Morin-Rivron, K. Simons, M. Masoodi, J. L. Sampaio, An automated shotgun lipidomics platform for high throughput, comprehensive, and quantitative analysis of blood plasma intact lipids. Eur J Lipid Sci Technol 117, 1540-1549 (2015).
79. G. Liebisch, M. Binder, R. Schifferer, T. Langmann, B. Schulz, G. Schmitz, High throughput quantification of cholesterol and cholesteryl ester by electrospray ionization tandem mass spectrometry (ESI-MS/MS). Biochim Biophys Acta 1761, 121-128 (2006).
80. R. Herzog, K. Schuhmann, D. Schwudke, J. L. Sampaio, S. R. Bornstein, M. Schroeder, A. Shevchenko, LipidXplorer: a software for consensual cross-platform lipidomics. PLoS One 7, e29851 (2012).
81. R. Herzog, D. Schwudke, K. Schuhmann, J. L. Sampaio, S. R. Bornstein, M. Schroeder, A. Shevchenko, A novel informatics concept for high-throughput shotgun lipidomics based on the molecular fragmentation query language. Genome Biol 12, R8 (2011).
82. R. Pamplona, M. Portero-Otin, D. Riba, C. Ruiz, J. Prat, M. J. Bellmunt, G. Barja, Mitochondrial membrane peroxidizability index is inversely related to maximum life span in mammals. J Lipid Res 39, 1989-1994 (1998).
83. R. C. Team, R: A language and environment for statistical computing. (2018).
84. H. Wickham, ggplot2: Elegant Graphics for Data Analysis.. Springer-Verlag New York https://ggplot2.tidyverse.org (2016).
85. M. Martin, Cutadept removes adapter sequences from high-throughput sequncing reads. EMBnet.journal 7, 2803-2809 (2011).
86. A. Dobin, C. A. Davis, F. Schlesinger, J. Drenkow, C. Zaleski, S. Jha, P. Batut, M. Chaisson, T. R. Gingeras, STAR: ultrafast universal RNA-seq aligner. Bioinformatics 29, 15-21 (2013).
87. S. Anders, P. T. Pyl, W. Huber, HTSeq--a Python framework to work with high-throughput sequencing data. Bioinformatics 31, 166-169 (2015).
88. S. Anders, W. Huber, Differential expression analysis for sequence count data. Genome Biol 11, R106 (2010).
89. A. Subramanian, P. Tamayo, V. K. Mootha, S. Mukherjee, B. L. Ebert, M. A. Gillette, A. Paulovich, S. L. Pomeroy, T. R. Golub, E. S. Lander, J. P. Mesirov, Gene set enrichment analysis: a knowledge-based approach for interpreting genome-wide expression profiles. Proc Natl Acad Sci U S A 102, 15545-15550 (2005).
90. M. Alhamdoosh, M. Ng, N. J. Wilson, J. M. Sheridan, H. Huynh, M. J. Wilson, M. E. Ritchie, Combining multiple tools outperforms individual methods in gene set enrichment analyses. Bioinformatics 33, 414-424 (2017).
91. J. Schindelin, I. Arganda-Carreras, E. Frise, V. Kaynig, M. Longair, T. Pietzsch, S. Preibisch, C. Rueden, S. Saalfeld, B. Schmid, J. Y. Tinevez, D. J. White, V. Hartenstein, K. Eliceiri, P. Tomancak, A. Cardona, Fiji: an open-source platform for biological-image analysis. Nat Methods 9, 676-682 (2012).
92. V. I. Alexaki, G. Fodelianaki, A. Neuwirth, C. Mund, A. Kourgiantaki, E. leronimaki, K. Lyroni, M. Troullinaki, C. Fujii, W. Kanczkowski, A. Ziogas, M. Peitzsch, S. Grossklaus, B. Sonnichsen, A. Gravanis, S. R. Bornstein, I. Charalampopoulos, C. Tsatsanis, T. Chavakis, DHEA inhibits acute microglia-mediated inflammation through activation of the TrkA-Akt1/2-CREB-Jmjd3 pathway. Mol Psychiatry 23, 1410-1420 (2018).
93. R. L. Erickson, C. A. Browne, I. Lucki, Hair corticosterone measurement in mouse models of type 1 and type 2 diabetes mellitus. Physiol Behav 178, 166-171 (2017).
94. K. Chapman, M. Holmes, J. Seckl, 11beta-hydroxysteroid dehydrogenases: intracellular gate-keepers of tissue glucocorticoid action. Physiol Rev 93, 1139-1206 (2013).

## Claims

1. An eicosapentaenoic acid (EPA) ester derivative for use in the prevention and/or treatment of obesity in a human subject.

2. The EPA ester derivative for use according to the preceding claim, wherein the obesity is central obesity.

3. The EPA ester derivative for use according to any one of the preceding claims, wherein the obesity is associated with cortisol levels above the average of healthy human subjects.

4. The EPA ester derivative for use according to any one of the preceding claims, wherein the subject suffers from depression.

5. The EPA ester derivative for use according to any one of the preceding claims, wherein the EPA ester derivative is eicosapentaenoic acid ethyl ester (icosapent ethyl).

6. The EPA ester derivative for use according to any one of the preceding claims, wherein the subject has above average levels of glucocorticoid, such as cortisol.

7. The EPA ester derivative for use according to any one of the preceding claims, wherein the subject has levels of cortisol (preferably cortisol levels in a hair sample, a blood sample, more preferably a plasma sample) above a reference value and/or above an average cortisol level in a healthy human population.

8. The EPA ester derivative for use according to any one of the preceding claims, wherein glucocorticoid levels, such as cortisol levels, are reduced in the patient upon treatment.

9. The EPA ester derivative for use according to any one of the preceding claims, wherein the EPA ester derivative inhibits the expression of fatty acid desaturase 2 (FADS2) in tissues, preferably adrenal cortex, in the patient upon treatment.

10. The EPA ester derivative for use according to any one of the preceding claims, wherein transcription of the fatty acid desaturase 2 (*FADS2*) gene is altered in the adrenal cortex upon treatment.

11. The EPA ester derivative for use according to any one of the preceding claims, wherein the cortisol and/or aldosterone levels are reduced in the patient upon treatment, without altering the adrenocorticotropic hormone (ACTH) plasma levels.

12. The EPA ester derivative for use according to any one of the preceding claims, wherein concentration of polyunsaturated fatty acids (PUFA) (preferably omega-3 fatty acids) is increased in the patient upon treatment.

13. The EPA ester derivative for use according to any one of the preceding claims, wherein the levels of pro-inflammatory lipid mediators are decreased in the patient upon treatment.

14. The EPA ester derivative for use according to any one of the preceding claims, wherein the patient has or is undergoing a combined (preferably simultaneous or sequential) treatment with one or more other active agents for treating obesity and/or other medical treatments for obesity, such as surgery.

15. A pharmaceutical composition comprising an EPA ester derivative with one or more pharmaceutical excipients for use according to any one of the preceding claims.
